# EUROPÄISCHE PATENTSCHRIFT

(11) **EP 3 110 383 B1**
(45) Veröffentlichungstag und Bekanntmachung des Hinweises auf die Patenterteilung: **04.04.2018**
(21) Anmeldenummer: 15705623.5
(22) Anmeldetag: 23.02.2015
(51) Int. Cl.: A61F 13/496, A61F 13/58, A61F 13/49, A61F 13/15

(54) **HYGIENEARTIKEL UND VERFAHREN ZUM HERSTELLEN EINES HYGIENEARTIKELS**
SANITARY ARTICLE AND METHOD FOR PRODUCING A SANITARY ARTICLE
ARTICLE D'HYGIÈNE ET PROCÉDÉ DE FABRICATION DUDIT ARTICLE D'HYGIÈNE

(30) Priorität: 28.02.2014 DE 102014203742
(43) Veröffentlichungstag der Anmeldung: 04.01.2017
(73) Patentinhaber: Paul Hartmann Aktiengesellschaft, 89522 Heidenheim (DE)
(72) Erfinder: GROENER, Rudolf, 89555 Soehnstetten (DE); KOCH, Christian, 89429 Bachhagel (DE); EILERS, Joerg, 89073 Ulm (DE); CIFRIC, Nedzad, 89542 Herbrechtingen (DE)
(74) Vertreter: DREISS Patentanwälte PartG mbB
(86) Internationale Anmeldenummer: PCT/EP2015/053718
(87) Internationale Veröffentlichungsnummer: WO 2015/128284

(56) Entgegenhaltungen:
- EP-A1- 1 228 741
- EP-A1- 2 221 034
- WO-A1-96/04874
- DE-A1-102012 208 393

## Beschreibung

Die Erfindung betrifft einen Hygieneartikel, wie insbesondere Windel, Höschenwindel, Inkontinenzvorlage, mit einer Produktlängsrichtung und einer Produktquerrichtung, mit einem Absorptionskörper und mit Beinöffnungen oder Beinausschnitte begrenzenden beidseitigen Randbereichen, wobei in Produktquerrichtung beidseits außerhalb des Absorptionskörpers und entlang zumindest eines Teils der beidseitigen Randbereiche langgestreckte Elastifizierungsmittel vorgesehen und zwischen Flachmaterialabschnitten des Hygieneartikels fixiert sind, so dass eine Elastifizierung entlang zumindest eines Teils der Beinöffnungen oder der Beinausschnitte resultiert, wobei die Elastifizierungsmittel entlang zumindest eines Teils der Beinöffnungen oder der Beinausschnitte bogenförmig gekrümmt verlaufen, und wobei ein körperzugewandter Flachmaterialabschnitt ein Vlies umfasst.

Bei Hygieneartikeln mit entlang zumindest eines Teils der Beinöffnungen oder der Beinausschnitte vorgesehenen Elastifizierungsmitteln, bei denen es sich typischerweise um fadenförmige oder auch bandförmige Elastifizierungsmittel handeln kann, besteht die Aufgabe, diese Elastifizierungsmittel prozesssicher zwischen Flachmaterialien des Hygieneartikels zu fixieren. Bei bogenförmig gekrümmtem Verlauf der Elastifizierungsmittel stellt sich dies regelmäßig schwieriger dar als bei exakt in Maschinenrichtung verlaufender Erstreckung der Elastifizierungsmittel in einer schnelllaufenden Maschine. Bogenförmig gekrümmte oder geschwungene Elastifizierungsmittel entlang der Beinöffnungen können durch einen insbesondere getakteten Sprühkleberaufrag auf das eine Flachmaterial oder auf beide Flachmaterialien, zwischen denen sie sandwichartig aufgenommen werden, fixiert werden. Die Verwendung von Sprühklebern birgt indessen Probleme innerhalb der Herstellungsmaschine, da nebelartig versprühter Kleber sowohl das Produkt als auch die Maschine oder Maschinenteile verunreinigt. Die Anwendung von Sprühkleber und insbesondere Spiralverklebung mittels Sprühverfahren unter Zuführung von heißer Luft verursacht Luftverwirbelung innerhalb der Maschine, was zu Verunreinigungen, beispielsweise der Umlenkrollen im Bereich des Sprühkopfs führt. Der Reinigungsaufwand der Maschine wird erhöht, und es kann zu Maschinenstillständen infolge von Bahnabrissen kommen. Bei getaktetem Sprühkleberauftrag kommt es infolge der Taktung zum Aufbau eines Überdrucks im Bereich des Sprühkopfs, der sich bei Öffnung des Sprühventils in einem überhöhten Kleberausstoß äußert. Es ist daher schwer, im getakteten Betrieb gleichförmigen Sprühkleberauftrag zu erreichen.

Ein weiteres Problem besteht darin, dass bei Kleberauftrag direkt auf thermoplastische Film- oder Folienmaterialien der Schmelzpunkt eines dünnen Films oder einer dünnen Folie nahe bei der Verarbeitungstemperatur des Klebers liegt. Dies bedingt die Gefahr des Anschmelzens der Kunststofffolie. Es kann zu Qualitätsmängeln im Produkt kommen. Auch wenn ein partielles Anschmelzen der Folie nicht zu Undichtigkeiten führt, so führt es doch zu bereichsweisen Verhärtungen der Folie, was als unangenehm empfunden werden kann. Bei Anwendung von Spiralverklebung kommt es stets zu Bereichen ohne Kleberauftrag, wo die Elastifizierungsmittel folglich unfixiert bleiben und in der Folge unbeabsichtigt verrutschen können. Die EP 1 228 741 A1 beschreibt eine Wegwerfwindel, die elastische Elemente enthält, die mit Beinlöchern verbunden sind und jeweils eine Vielzahl von elastischen Elementen aufweisen.

Der vorliegenden Erfindung liegt die Aufgabe zugrunde, einen Hygieneartikel sowie ein Verfahren zu seiner Herstellung anzugeben, bei denen die vorstehend geschilderten Probleme nicht oder in geringerem Maße auftreten.

Diese Aufgabe wird erfindungsgemäß durch einen Hygieneartikel der eingangs genannten Art gelöst, der dadurch gekennzeichnet ist, dass das Vlies im Bereich der beidseitigen Elastifizierungsmittel und zu deren Fixierung auf seiner körperabgewandten Seite einen streifenförmigen, vollflächig aufgebrachten und in der Produktlängsrichtung erstreckten ersten Kleberauftrag aufweist und dass das Vlies im Bereich der beidseitigen Elastifizierungsmittel und zu deren Fixierung auf seiner körperabgewandten Seite beidseits in Produktquerrichtung außerhalb des ersten Kleberauftrags einen streifenförmigen, vollflächig aufgebrachten und in der Produktlängsrichtung erstreckten zweiten Kleberauftrag aufweist, der in Produktquerrichtung mit dem ersten Kleberauftrag teilweise überlappt oder unmittelbar an den ersten Kleberauftrag angrenzt oder von dem ersten Kleberauftrag in Produktquerrichtung nach außerhalb beabstandet ist, und dass der zweite Kleberauftrag in Produktlängsrichtung nach vorn und in Produktlängsrichtung nach hinten jeweils kürzer ist als der erste Kleberauftrag, also vor diesem endet, und infolge seitlicher Konturierung der beidseitigen Randbereiche zur Bildung der Beinöffnungen oder Beinausschnitte auch unterbrochen oder konturiert sein kann, und dass die langgestreckten Elastifizierungsmittel durch den ersten und den zweiten Kleberauftrag zwischen dem Vlies und einem gegenüber dem Vlies körperabgewandten Flachmaterialabschnitt fixiert sind.

Es wird also von einem Sprühkleberauftrag Abstand genommen, und es wird stattdessen ein streifenförmiger, vollflächig aufgebrachter und in der Produktlängsrichtung erstreckter erster und zweiter Kleberauftrag vorgesehen. Unter einem streifenförmig und vollflächig aufgebrachten Kleberauftrag wird eine auch als Kontaktbeleimung bezeichnete Aufbringung von schmelzflüssigem Klebermaterial verstanden. Das flüssige Klebermaterial wird hierbei aus einer wenigstens nahezu bei dem Vlies angeordneten typischerweise schlitzförmigen Kleberaustrittsöffnung einer Beleimungsvorrichtung hindurch direkt auf das Vlies aufgebracht. Es wird also nicht über Düsen versprüht, sondern gewissermaßen als Film direkt streifenförmig und vollflächig auf das Vliesmaterial aufgebracht.

Der zweite Kleberauftrag kann in Produktquerrichtung mit dem ersten Kleberauftrag insbesondere höchstens 10 mm, insbesondere höchstens 7 mm und weiter insbesondere höchstens 5 mm überlappen oder unmittelbar an den ersten Kleberauftrag angrenzen oder höchstens 10 mm, insbesondere höchstens 7 mm, insbesondere höchstens 5 mm von dem ersten Kleberauftrag beabstandet sein. Dadurch, dass der zweite Kleberauftrag in Produktlängsrichtung nach vorn und nach hinten jeweils kürzer ist als der erste Kleberauftrag, wird ausgehend von einer Längsmittelachse des Hygieneartikels in Produktquerrichtung jeweils außerhalb des ersten Kleberauftrags ein kleberfreier Bereich gewissermaßen in gedachter Verlängerung des streifenförmigen zweiten Kleberauftrags gebildet. Wenn in diesem Bereich in der Produktlängsrichtung bei der Herstellung des Hygieneartikels die Elastifizierungsmittel geführt werden, so werden sie in diesem Bereich nicht mit den Flachmaterialien fixiert. Sie können dann durch an sich übliche Techniken hinsichtlich ihrer elastifizierenden Wirkung benommen, also deelastifiziert werden, insbesondere geschnitten werden oder durch Anwendung von Ultraschall, Laser oder in sonstiger Weise deelastifziert werden.

In dem Bereich, in dem die Elastifizierungsmittel in dem ersten und zweiten Kleberauftrag verlaufen und erstreckt sind, werden sie hingegen prozesssicher fixiert. Dabei erstrecken sich die Elastifizierungsmittel insbesondere mit ihrem bogenförmig gekrümmten Verlauf in dem ersten Kleberauftrag und gelangen von dort entlang ihrer weiteren Erstreckung in den zweiten Kleberauftrag. Von dort können sie sich in den vorstehend erwähnten kleberfreien Bereich erstrecken und dort insbesondere linear der weiteren Produktlängsrichtung nach hinten und vorn folgen.

Die Elastifizierungsmittel können also derart geführt und zwischen die Flachmaterialien eingebracht sein, dass der bogenförmig gekrümmte Verlauf der beidseitigen Elastifizierungsmittel zumindest durch den ersten Kleberauftrag verläuft und fixiert ist.

Nach einer weiteren Ausführungsform der Erfindung verläuft ein an den bogenförmigen Verlauf der Elastifizierungsmittel anschließender linearer oder gerader Verlauf der Elastifizierungsmittel nur durch den zweiten Kleberauftrag und ist durch diesen fixiert.

Als Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt. Die Elastifizierungsmittel haben vorzugsweise eine Stärke von 300-1500 dtex, insbesondere von 500-1200 dtex, weiter insbesondere von 500-900 dtex. Die Elastifizierungsmittel werden vorzugsweise unter einer Vorspannung von 1,5-6,0, insbesondere von 2,5-4.,5 zwischen dem Vlies und dem körperabgewandten Flachmaterialabschnitt fixiert.

Es erweist sich als vorteilhaft, wenn der erste Kleberauftrag in Produktquerrichtung eine Breite von wenigstens 20 mm, insbesondere von wenigstens 25 mm, und von höchstens 40 mm, insbesondere von höchstens 35 mm aufweist.

Weiter erweist es sich als vorteilhaft, wenn der erste und/oder der zweite Kleberauftrag ein Flächengewicht von wenigstens 1 g/m², insbesondere von wenigstens 2 g/m², insbesondere von höchstens 20 g/m², insbesondere von höchstens 10 g/m², insbesondere von höchstens 7 g/m² und weitere insbesondere von 2 - 5 g/m² aufweist.

Weiter erweist es sich als vorteilhaft, dass der zweite Kleberauftrag in Produktquerrichtung eine Breite von wenigstens 5 mm, insbesondere von wenigstens 10 mm, insbesondere von wenigstens 15 mm und von höchstens 30 mm, insbesondere von höchstens 25 mm, und weiter insbesondere von höchstens 20 mm aufweist.

Es erweist sich als besonders vorteilhaft, wenn der zweite Kleberauftrag in Produktlängsrichtung nach vorn und in Produktlängsrichtung nach hinten jeweils um eine Distanz (D) kürzer ist, die wenigstens 15 %, insbesondere wenigstens 20 % und höchstens 35 %, insbesondere höchstens 30 % der Gesamtlänge (L₁) des ersten Kleberauftrags in Produktlängsrichtung beträgt. Diese Distanz, um die der zweite Kleberauftrag jeweils hinten und vorn kürzer ist als der erste Kleberauftrag bildet den vorerwähnten kleberfreien Bereich beidseits außerhalb des ersten Kleberauftrags.

In Weiterbildung der Erfindung erweist es sich als vorteilhaft, wenn das Vlies auf seiner körperabgewandten Seite und beidseits in Produktquerrichtung außerhalb des zweiten Kleberauftrags und außerhalb der beidseitigen Elastifizierungsmittel einen streifenförmigen, vollflächig aufgebrachten und in der Produktlängsrichtung erstreckten dritten Kleberauftrag aufweist. Der dritte Kleberauftrag überlappt in Produktquerrichtung dabei teilweise mit dem zweiten Kleberauftrag oder ist unmittelbar an den zweiten Kleberauftrag angrenzend oder ist von dem zweiten Kleberauftrag in Produktquerrichtung nach außerhalb beabstandet. Der dritte Kleberauftrag kann in Produktquerrichtung insbesondere höchstens bis zur Hälfte der Breite des dritten Kleberauftrags vom zweiten Kleberauftrag überlappt sein oder unmittelbar an den zweiten Kleberauftrag angrenzen oder mit einem Abstand von höchstens der Hälfte der Breite des dritten Kleberauftrags vom zweiten Kleberauftrag in Produktquerrichtung nach außerhalb beabstandet sein.

Der dritte Kleberauftrag weist dabei insbesondere eine Breite von 2-10 mm auf. Insbesondere weist der dritte Kleberauftrag ein Flächengewicht von wenigstens 1 g/m², insbesondere von wenigstens 2 g/m², insbesondere von höchstens 20 g/m², insbesondere von höchstens 10 g/m ² und weitere insbesondere von 4 - 8 g/m² auf.

In Weiterbildung dieses Erfindungsgedankens erweist es sich als vorteilhaft, dass in Produktquerrichtung zwischen dem ersten und dem dritten Kleberauftrag und in Produktlängsrichtung hinten und vorn ein kanalförmiger kleberfreier Abschnitt zwischen dem Vlies und dem dazu körperabgewandt angeordneten Flachmaterialabschnitt gebildet ist. Dieser kleberfreie Abschnitt ist derjenige schon vorerwähnte Bereich, wo der zweite Kleberauftrag nicht vorgesehen ist, weil der zweite Kleberauftrag in Produktlängsrichtung nach vorn und nach hinten vor dem ersten Kleberauftrag endet, also kürzer als dieser ausgebildet ist.

Es erweist sich im Hinblick auf den dritten Kleberauftrag als vorteilhaft, wenn der dritte Kleberauftrag zumindest abschnittsweise randbündig mit Längsrändern des Vlieses ausgebildet ist. Auf diese Weise kann eine randbündige Fügung, d. h. Verklebung des Vlieses mit dem diesbezüglich körperabgewandten Flachmaterial erreicht werden. Hierdurch kann eine weitere Barriere für Flüssigkeitstransport in Produktquerrichtung erreicht werden. Es wird so kapillarer Flüssigkeitsleitung in Produktquerrichtung weiter entgegengewirkt.

Die erwähnten beidseitigen Beinöffnungen oder Beinausschnitte können grundsätzlich durch linear, also gerade erstreckte Ränder oder durch bogenförmig konturierte Ränder begrenzt werden. Beides wird typischerweise durch seitliche Konturierung der Flachmaterialbahnen, insbesondere durch Schneidwalzen realisiert. Es erweist sich diesbezüglich als vorteilhaft, wenn der zweite Kleberauftrag, und insbesondere zusätzlich der erste Kleberauftrag durch die seitliche Konturierung der beidseitigen Randbereiche zur Bildung von Beinöffnungen oder Beinausschnitten abschnittsweise randbündig mit Längsrändern des Vlieses ausgebildet sind. Dies ergibt sich, wenn der beidseitige Konturschnitt zur Bildung der Beinöffnungen oder Beinausschnitte durch den ersten und insbesondere auch den zweiten Kleberauftrag verläuft. Auch hierdurch wird eine randbündige Verklebung des Vlieses mit dem hierzu körperabgewandt angeordneten Flachmaterial erreicht, was auch in diesem Bereich dem unerwünschten Flüssigkeitstransport in Produktquerrichtung entgegenwirkt.

Es kann sich auch als vorteilhaft erweisen, wenn sich der erste und der zweite Kleberauftrag durch unterschiedliche Klebermaterialien und/oder durch unterschiedliches Flächengewicht voneinander unterscheiden. Es können so die spezifischen Anforderungen besser erfüllt werden. Beispielsweise erfordert die Einbettung von bogenförmig gekrümmten Elastifizierungsmitteln vorzugsweise einen Kleber mit stärkerer Haftkraft und/oder höherem Flächengewicht als dies zur Fixierung von linear erstreckten Elastifizierungsmitteln erforderlich ist. Es kann auch zu beachten sein, dass der erste und der zweite Kleberauftrag eine duale Funktion haben, sie fixieren die Elastifizierungsmittel und verbinden das körperzugewandte Vlies mit dem diesbezüglich körperabgewandten Flachmaterial.

Es erweist sich weiter als vorteilhaft, wenn das Vlies auf seiner körperabgewandten Seite und in Produktquerrichtung zwischen dem beidseitigen ersten Kleberauftrag vollflächig aufgebrachte und in der Produktlängsrichtung und vorzugsweise parallel zueinander erstreckte Kleberspuren als vierten Kleberauftrag aufweist. Auch das Klebermaterial und/oder das Flächengewicht des dritten oder vierten Kleberauftrags können sich sowohl voneinander als auch von denjenigen des ersten oder zweiten Kleberauftrags unterscheiden. Die Funktion des dritten und vierten Kleberauftrags unterscheidet sich jedenfalls insoweit von derjenigen des ersten und zweiten Kleberauftrags als dass sie nicht zur Fixierung der Elastifizierungsmittel vorgesehen sind, sondern im Wesentlichen zur Fixierung des körperzugewandten Vlieses mit dem diesbezüglich körperabgewandten Flachmaterialabschnitt oder zur Fixierung von zwischen dem körperzugewandten Vlies und dem körperabgewandten Flachmaterialabschnitt vorhandenen Komponenten, wie beispielsweise eines Absorptionskörpers, dienen.

Hierbei erweist es sich als vorteilhaft, wenn die Breite der Kleberspuren des vierten Kleberauftrags 1 - 5 mm, insbesondere 2 - 5 mm, insbesondere 2 - 4 mm beträgt, und/oder dass der Abstand der Kleberspuren des vierten Kleberauftrags voneinander 2-20 mm, insbesondere 2 - 10 mm, insbesondere 2 - 6 mm, insbesondere 2 - 4 mm beträgt.

Für den ersten, zweiten, dritten und/oder vierten Kleberauftrag wird vorzugsweise ein Hotmeltkleber, weiter insbesondere ein hydrophober Hotmeltkleber eingesetzt.

Der körperzugewandte Flachmaterialabschnitt umfasst erfindungsgemäß ein Vlies. Weiter vorteilhaft kann der körperzugewandte Flachmaterialabschnitt abschnittsweise oder auch lagenweise weitere Materialien aufweisen, wie beispielsweise Lochfolien.

Es erweist sich insbesondere als vorteilhaft, wenn das Vlies ein Spunbondmaterial, ein Laminat aus Spunbondlagen (S) und Meltblownlagen (M) oder ein Stapelfaservliesmaterial oder Kombinationen davon umfasst und insbesondere ein Flächengewicht von mindestens 6 g/m², insbesondere von mindestens 10 g/m², insbesondere von höchstens 30 g/m², insbesondere von höchstens 20 g/m² aufweist. Insbesondere vorteilhaft bildet das Vlies eine körperkontaktierende Topsheet-Lage des Hygieneartikels..

Der bezüglich dem Vlieskörper abgewandte Flachmaterialabschnitt, welcher zusammen mit dem Vlies und dem Kleber die Elastifizierungsmittel fixiert, kann in vorteilhafter Weise eine thermoplastische Folien- oder Filmschicht, insbesondere eine mikroporöse Folie oder ein Vlies-Folien-Laminat umfassen.

Es erweist sich erfindungsgemäß als besonders vorteilhaft, wenn zur Fixierung der Elastifizierungsmittel nur der vorerwähnte erste und zweite Kleberauftrag verwendet wird, wenn also in dem Bereich der Elastifizierungsmittel keine direkte Beleimung des körperabgewandten Flachmaterialabschnitts, sondern nur des diesbezüglich körperzugewandten Vlieses erfolgt. Auf diese Weise wird dann der zumeist aus einer thermoplastischen Folien- oder Filmschicht gebildete körperabgewandte Flachmaterialabschnitt vor heißem Klebermaterial besser geschützt, so dass die eingangs geschilderte Problematik des Durchschmelzens von thermoplastischem Material besser verhindert werden kann. Als besonders vorteilhaft erweist es sich, wenn auf den thermoplastischen Fiachmaterialabschnitt an keiner Stelle direkt schmelzflüssiges Klebermaterial aus einer Auftragsvorrichtung aufgebracht wird, sondern erst im Anschluss an die Aufbringung auf andere Flachmaterialien, wie z. B. das körperzugewandte Vlies. Im Hinblick auf die Verschmutzungsproblematik innerhalb der Maschine erweist es sich auch als vorteilhaft, wenn keine Sprühbeleimung der Elastifizierungsmittel vorgenommen wird, zumal dies infolge der flächenhaften Kontaktbeleimung in Form des ersten und zweiten Kleberauftrags auch nicht mehr erforderlich ist.

Weiter kann es sich als vorteilhaft erweisen, wenn sich der erste und/oder der dritte und/oder der vierte Kleberauftrag in Produktlängsrichtung bis zu Längsenden des Hygieneartikels erstrecken. In diesem Fall können die betreffenden Kleberaufträge in der Maschinenrichtung endlos appliziert werden, was sich herstellungstechnisch stets als vorteilhaft erweist. In diesem Fall bilden das in dem körperzugewandten Flachmaterialabschnitt umfasste Vlies und der demgegenüber körperabgewandt angeordnete Flachmaterialabschnitt zugleich die Hüllen- oder Chassismaterialien des Hygieneartikels, die den Hygieneartikel im eben ausgebreiteten, also flächenhaft ausgedehnten Zustand definieren und begrenzen (von etwa überstehenden Verschlusslaschen abgesehen).

Eine andere Ausführungsform ist gekennzeichnet durch eine den Absorptionskörper umfassende Schichtstruktur, welche die beidseitigen Randbereiche bildet, in denen die langgestreckten Elastifizierungsmittel entlang zumindest eines Teils der Beinöffnungen oder Beinausschnitte vorgesehen sind. Diese den Absorptionskörper umfassende Schichtstruktur kann dabei mit weiteren chassisbildenden Komponenten des Hygieneartikels verbunden bzw. durch diese in der vorgenannten ebenen Projektion ergänzt werden. Es wäre in diesem Sinne denkbar, dass lediglich die den Absorptionskörper umfassende Schichtstruktur in der vorausgehend genannten Weise mit erstem und zweitem Kleberauftrag und gegebenenfalls drittem und viertem Kleberauftrag ausgebildet ist und separat endlos gefertigt wird. Solchenfalls können diese Schichtstrukturen durch Vereinzelung quer zur Fertigungsrichtung einzeln erhalten und weiteren Komponenten in einer schnelllaufenden Herstellungsmaschine zugeführt werden. In Weiterführung dieses Erfindungsgedankens wird Schutz beansprucht für einen Hygieneartikel mit einer einen Absorptionskörper umfassenden Schichtstruktur, an der seitliche Abschnitte angebracht sind, insbesondere seitliche Abschnitte mit Verschlusslaschen für die Bereitstellung als offen- und/oder schließbaren Hygieneartikel. In einer anderen Weiterführung dieses Erfindungsgedankens wird Schutz beansprucht für einen Hygieneartikel mit einem vorderen Bauchabschnitt und einem hinteren Rückenabschnitt, die von separaten und in Produktlängsrichtung voneinander beabstandeten Komponenten gebildet sind, jedoch zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem den Absorptionskörper aufweisenden Schrittabschnitt, der sich in der Produktlängsrichtung zwischen Bauchabschnitt und Rückenabschnitt erstreckt und diese überbrückt und an den Bauchabschnitt und an den Rückenabschnitt in einem jeweiligen Überlappungsbereich unlösbar angefügt ist, wobei der Bauchabschnitt, der Rückenabschnitt und der Schrittabschnitt gemeinsam die Beinöffnungen begrenzen, wobei in dem Bauchabschnitt und in dem Rückenabschnitt in Produktquerrichtung verlaufende weitere Elastifizierungsmittel vorgesehen sind, die den Bauchabschnitt und den Rückenabschnitt flächenhaft elastifizieren, und wobei der Schrittabschnitt die den Absorptionskörper umfassende Schichtstruktur bildet. Dieser Hygieneartikel ist also aus drei Komponenten, nämlich dem vorderen Bauchabschnitt, dem hinteren Rückenabschnitt und dem Schrittabschnitt gebildet, wobei letzterer als separat gefertigte Einheit erfindungsgemäß, d. h. mit erstem und zweitem Kleberauftrag in der vorausgehend beschriebenen Weise ausgebildet ist und insbesondere und vorteilhafterweise auch den dritten und/oder vierten Kleberauftrag aufweist.

Der Schrittabschnitt, d. h. die Schichtstruktur ist dabei in einem jeweiligen Überlappungsbereich mit dem Bauchabschnitt und dem Rückenabschnitt insbesondere mittels Kleber fixiert. Die Art und Weise, Anordnung und Ausbildung der Kleberfixierung ist in DE 102007055524 A1 beschrieben, und es wird der diesbezügliche Inhalt dieser DE 102007055524 A1 durch Bezugnahme in die vorliegende Anmeldung einbezogen. Diese Art und Weise, Anordnung und Ausbildung der Kleberfixierung ist bevorzugt, aber nicht zwingend. Weiter vorzugsweise kann die Anordnung und Ausbildung der Kleberfixierung wie in DE 102012208393 A1 beschrieben, erfolgen und es wird der diesbezügliche Inhalt dieser DE 102012208393 A1 durch Bezugnahme in die vorliegende Anmeldung einbezogen.

Es kann sich auch als vorteilhaft erweisen, wenn sich die weiteren Elastifizierungsmittel in dem Bauchabschnitt und dem Rückenabschnitt in einem Abstand voneinander und parallel zueinander in Produktquerrichtung oder Hüftumfangsrichtung des Hygieneartikels erstrecken und so den Bauchabschnitt und den Rückenabschnitt flächenhaft elastifizieren. Weiter kann es sich als vorteilhaft erweisen, wenn in einem schrittseitigen und den Beinöffnungen zugewandten Bereich des Bauchabschnitts und des Rückenabschnitts Elastifizierungsmittel vorgesehen sind, die sich insbesondere ausgehend von den beiden Seitennahtbereichen in Richtung auf eine Längsmittelachse des Inkontinenzartikels erstrecken und sich dabei bis in den Überlappungsbereich von Schrittabschnitt und Bauchabschnitt bzw. von Schrittabschnitt und Rückenabschnitt erstrecken, wobei sie dort insbesondere ihrer elastifizierenden Wirkung benommen sein können, insbesondere geschnitten sein können.

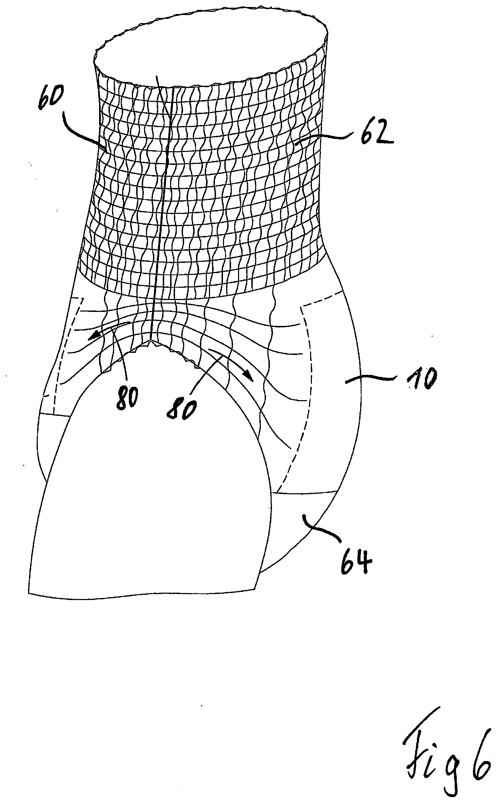

Die Ausbildung und Anordnung der in Hüftumfangsrichtung verlaufenden Elastifizierungsmittel und der insbesondere in einem schrittseitigen und den Beinöffnungen zugewandten Bereich des Bauchabschnitts und des Rückenabschnitts vorgesehenen Elastifizierungsmittel sind in DE 102007055524 A1 beschrieben, so dass der diesbezügliche Inhalt von DE 102007055524 A1 durch Bezugnahme in die vorliegende Anmeldung einbezogen wird. Dies gilt auch für den Spannungsverlauf der in einem schrittseitigen und den Beinöffnungen zugewandten Bereich des Bauchabschnitts und des Rückenabschnitts vorgesehenen Elastifizierungsmittel, insbesondere durch abnehmende Flächenrückstellkräfte in den den Beinöffnungen zugewandten Bereichen von Bauchabschnitt bzw. Rückenabschnitt (so wie in Fig.6 in Richtung der Pfeile 80 angedeutet ist).

Es erweist sich weiter als vorteilhaft, wenn sich der erste und/oder der dritte und/oder der vierte Kleberauftrag in Produktlängsrichtung bis zu Längsenden der den Absorptionskörper umfassenden Schichtstruktur erstreckt, also bis zu Längsenden des Schrittabschnitts erstreckt.

Gegenstand der Erfindung ist auch ein Verfahren zum Herstellen eines Hygieneartikels oder einer einen Absorptionskörper umfassenden Schichtstruktur eines Hygieneartikels oder im Besonderen ein Verfahren zum Fixieren von langgestreckten Elastifizierungsmitteln im Zuge der Herstellung eines Hygieneartikels mit den Merkmalen des Anspruchs 18. Es wird also ein Verfahren mit den folgenden Merkmalen vorgeschlagen:
- Zuführen eines den körperzugewandten Flachmaterialabschnitt bildenden endlosen Vlieses in einer Maschinenrichtung, die der späteren Produktlängsrichtung entspricht,
- kontinuierliches vollflächiges Aufbringen des ersten Kleberauftrags auf die körperabgewandte Seite des Vlieses in den beidseitigen Randbereichen in der Maschinenrichtung,
- getaktetes vollflächiges Aufbringen des zweiten Kleberauftrags in der Maschinenrichtung auf die körperabgewandte Seite des Vlieses in den beidseitigen Randbereichen in Produktquerrichtung außerhalb des ersten Kleberauftrags, derart, dass der zweite Kleberauftrag in Produktquerrichtung mit dem ersten Kleberauftrag teilweise überlappt oder unmittelbar an den ersten Kleberauftrag angrenzt oder von dem ersten Kleberauftrag in Produktquerrichtung beabstandet ist, wobei der zweite Kleberauftrag derart getaktet aufgebracht wird, dass er in Produktlängsrichtung nach vorn und hinten kürzer ist als der erste Kleberauftrag, also vor diesem endet,
- Zuführen einer den körperäbgewandten Flachmaterialabschnitt bildenden endlosen Flachmaterialbahn in der Maschinenrichtung,
- Zuführen der beidseitigen Elastifizierungsmittel in der Maschinenrichtung zwischen das Vlies und die körperabgewandte Flachmaterialbahn zumindest abschnittsweise im Bereich des ersten und des zweiten Kleberauftrags,
- Zusammenbringen des endlosen Vlieses und der körperabgewandten Flachmaterialbahn unter Zwischenanordnung der Elastifizierungsmittel und Fixieren der Elastifizierungsmittel durch den ersten und zweiten Kleberauftrag,
- Deelastifizieren der Elastifizierungsmittel in einem im Zuge der Taktung des zweiten Kleberauftrags in Produktlängsrichtung kleberfrei verbliebenen Bereich.

Das vorgenannte Vlies wird in Form einer in der Maschinenrichtung endlosen Vliesbahn entsprechend der den körperabgewandten Flachmaterialabschnitt bildenden Flachmaterialbahn zugeführt. Der jeweilige vollflächige Kleberauftrag wird wiederum wie eingangs beschrieben als eine Kontaktbeleimung aufgebracht. Die durch das erfindungsgemäße Verfahren erzielten Vorteile wurden bereits eingangs im Zusammenhang mit dem erfindungsgemäßen Hygieneartikel erörtert, so dass hierauf verwiesen wird. Es sei noch erwähnt, dass zum Deelastifizieren der Elastifizierungsmittel bei einer vorteilhaften Verfahrensführung ein jeweiliges Elastifizierungsmittel beispielsweise nur an einer einzigen Stelle getrennt zu werden braucht, so dass es sich über die Länge des kleberfrei verbliebenen Bereichs ungehindert zusammenziehen kann und auf diese Weise seine elastifizierende Wirkung auf die Chassismaterialien verliert, da es mit diesen im besagten Bereich ja nicht klebend verbunden ist. Hierbei erweist es sich als vorteilhaft, wenn dieser Bereich in Produktquerrichtung einerseits durch den ersten Kleberauftrag und nach außen hin durch den dritten Kleberauftrag begrenzt ist. Es wird auf diese Weise eine Art Kanal für die Elastifizierungsmittel geschaffen, der in Produktlängsrichtung erstreckt ist.

Das Verfahren ist vorteilhafterweise weiter gekennzeichnet durch vollflächiges Aufbringen eines dritten Kleberauftrags in der Maschinenrichtung auf die körperabgewandte Seite des Vlieses in den beidseitigen Randbereichen in Produktquerrichtung außerhalb des zweiten Kleberauftrags und außerhalb der beidseitigen Elastifizierungsmittel. Das Aufbringen des dritten Kleberauftrag ist dabei derart, dass der dritte Kleberauftrag sich in Produktquerrichtung dabei teilweise mit dem zweiten Kleberauftrag überlappt oder unmittelbar an den zweiten Kleberauftrag angrenzend ist oder von dem zweiten Kleberauftrag in Produktquerrichtung nach außerhalb beabstandet ist. Beim Aufbringen des dritten Kleberauftrags wird der dritte Kleberauftrag in Produktquerrichtung insbesondere höchstens bis zur Hälfte der Breite des dritten Kleberauftrags vom zweiten Kleberauftrag überlappt oder grenzt unmittelbar an den zweiten Kleberauftrag an oder ist mit einem Abstand von höchstens der Hälfte der Breite des dritten Kleberauftrags vom zweiten Kleberauftrag in Produktquerrichtung nach außerhalb beabstandet.

Das Verfahren ist vorteilhafterweise weiter gekennzeichnet durch vollflächiges Aufbringen eines vierten Kleberauftrags in der Maschinenrichtung auf die körperabgewandte Seite des Vlieses auf einen Bereich in Produktquerrichtung zwischen dem beidseitigen ersten Kleberauftrag in Form von nebeneinander erstreckten Kleberspuren, insbesondere mit einer Breite von 1 - 5 mm, insbesondere 2 - 5 mm, insbesondere 2-4 mm und in einem Abstand der Kleberspuren voneinander von 2 - 20 mm, insbesondere 2 - 10 mm, insbesondere 2 - 6 mm, insbesondere 2 - 4 mm.

Hierbei erweist es sich auch als vorteilhaft, wenn der dritte Kleberauftrag und/oder der vierte Kleberauftrag in der Maschinenrichtung kontinuierlich aufgebracht werden.

Es erweist sich auch als vorteilhaft, wenn zur Fixierung der Elastifizierungsmittel nur der erste und der zweite Kleberauftrag verwendet werden und die körperabgewandte Flachmaterialbahn unbeschichtet bleibt in diesem Bereich.

Es wird weiter vorgeschlagen, dass zur Aufbringung des ersten und des zweiten Kleberauftrags, insbesondere auch zur Aufbringung des dritten und/oder vierten Kleberauftrags, eine Kleberaufträgsvorrichtung verwendet wird, welche im nächsten Kontakt zum Vlies aus insbesondere schlitzförmigen Kleberaustrittsöffnungen Klebermaterial abgibt. Es erweist sich hierbei als vorteilhaft, wenn die Kleberauftragvorrichtung eine Förderdrucksteuer- und vorzugsweise Förderdruckregeleinrichtung umfasst. Auf diese Weise kann ein getakteter Kleberauftrag realisiert werden, ohne dass weitere mechanische Stellkomponenten verwendet werden müssen, die beispielsweise einen Kleberauftragskopf abheben müssen. Die Kleberauftragsvorrichtung kann für die einzelne oder parallele Aufbringung des ersten, zweiten, dritten und/oder vierten Kleberauftrags vorgesehen sein. Vorzugsweise ermöglicht die Kleberauftragsvorrichtung die zeitgleiche und parallele Aufbringung zumindest des ersten und dritten und/oder vierten Kleberauftrages.

Zur Ausbildung der Beinöffnungen oder Beinausschnitte erweist es sich als vorteilhaft, wenn nach Zusammenbringen des Vlieses und der körperabgewandten Flachmaterialbahn beidseits in der Maschinenrichtung geführte Konturschnitte angebracht werden, um die Beinöffnungen oder die Beinausschnitte zu bilden.

Weiter erweist es sich als vorteilhaft, wenn quer zur Maschinenrichtung ein Vereinzelungsschritt zur Bildung einzelner Hygieneartikel ausgeführt wird. Insbesondere erweist es sich als vorteilhaft, wenn das Deelastifizieren der Elastifizierungsmittel zusammen in einem quer zur Maschinenrichtung ausgeführten Vereinzelungsschritt zur Bildung einzelner Hygieneartikel ausgeführt wird.

Weiter erweist es sich als vorteilhaft, wenn mit dem erfindungsgemäßen Verfahren eine endlose Bahn von jeweils einen Absorptionskörper umfassenden Schichtstrukturen gefertigt wird, die die körperabgewandte Flachmaterialbahn und das körperzugewandte Vlies und eine endlose Folge von in Maschinenrichtung aufeinander geförderten Absorptionskörpern umfasst, die zwischen die körperabgewandte Flachmaterialbahn und das körperzugewandte Vlies gefördert und angeordnet werden, und dass quer zur Maschinenrichtung ein Vereinzelungsschnitt zur Bildung der Schichtstrukturen ausgeführt wird, und dass an die vereinzelten Schichtstrukturen seitliche Abschnitte, insbesondere seitliche Abschnitte mit Verschlusslaschen für die Bereitstellung als offen- und/oder schließbarer Hygieneartikel, angebracht werden.

Weiter erweist es sich als vorteilhaft, wenn mit dem erfindungsgemäßen Verfahren eine endlose Bahn von jeweils einen Absorptionskörper umfassenden Schichtstrukturen gefertigt wird, die die körperabgewandte Flachmaterialbahn und das körperzugewandte Vlies und eine endlose Folge von in Maschinenrichtung aufeinander geförderten Absorptionskörpern umfasst, die zwischen die körperabgewandte Flachmaterialbahn und das körperzugewandte Vlies gefördert und angeordnet werden, und dass quer zur Maschinenrichtung ein Vereinzelungsschnitt zur Bildung der Schichtstrukturen ausgeführt wird, dass die vereinzelten Schichtstrukturen um 90° gedreht und in überbrückende Anordnung mit einer Bauchabschnittbahn und einer Rückenabschnittbahn gebracht werden und im Überlappungsbereich mit der Bauchabschnittbahn und der Rückenabschnittbahn unlösbar fixiert werden.

Weitere Einzelheiten, Merkmale und Vorteile der Erfindung ergeben sich aus den beigefügten Patentansprüchen und aus der zeichnerischen Darstellung und nachfolgenden Beschreibung des erfindungsgemäßen Verfahrens und erfindungsgemäßer Hygieneartikel.

In der Zeichnung zeigt:
Figuren 1 und 2 schematische Ansichten zur Verdeutlichung des erfindungsgemäßen Herstellungsverfahrens;
Figur 3 eine Draufsicht auf eine erfindungsgemäße Schichtstruktur (schematisch zur Darstellung des streifenförmigen Kleberauftrags);
Figuren 4 a-c eine Draufsicht auf eine weitere Ausführungsform einer erfindungsgemäßen Schichtstruktur mit zwei Schnittansichten (schematisch zur Darstellung des streifenförmigen Kleberauftrags);
Figur 5 eine Draufsicht auf einen erfindungsgemäßen Hygieneartikel mit einem Schrittabschnitt entsprechend der Schichtstruktur nach Figur 3;
Figur 6 eine perspektivische Ansicht des Hygieneartikels nach Fig.5;
Figur 7 eine Draufsicht auf einen weiteren erfindungsgemäßen Hygieneartikel mit einem Schrittabschnitt entsprechend der Schichtstruktur nach Figur 4.

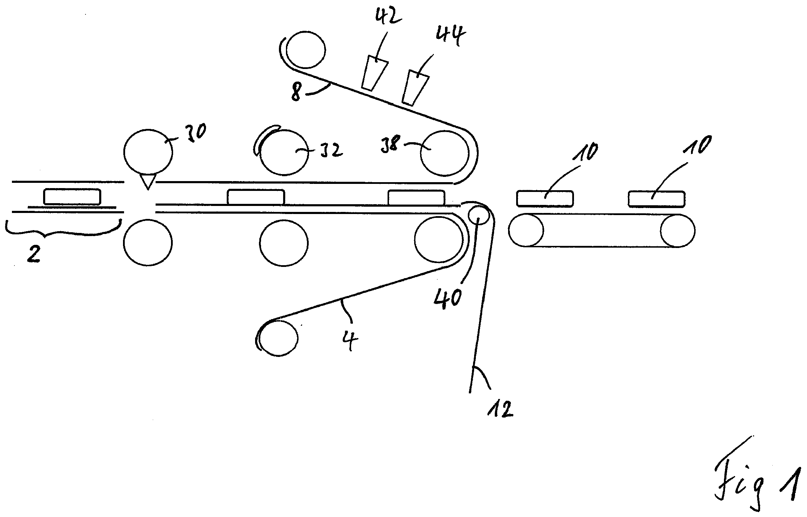

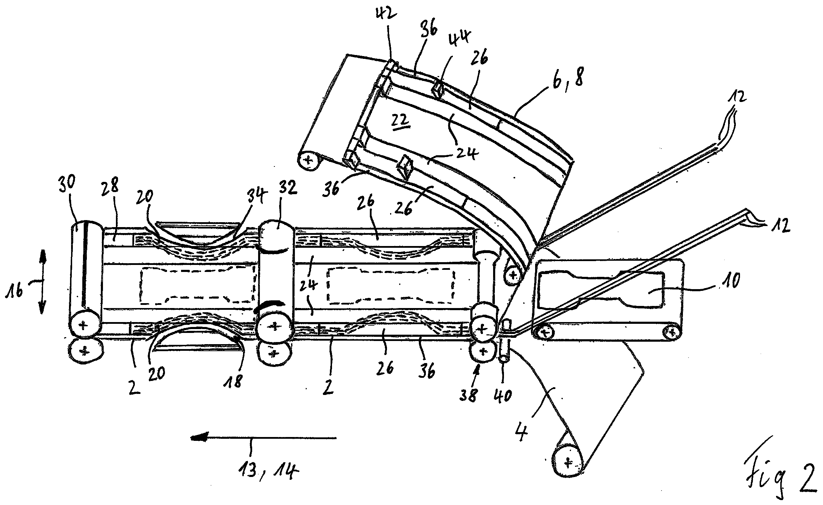

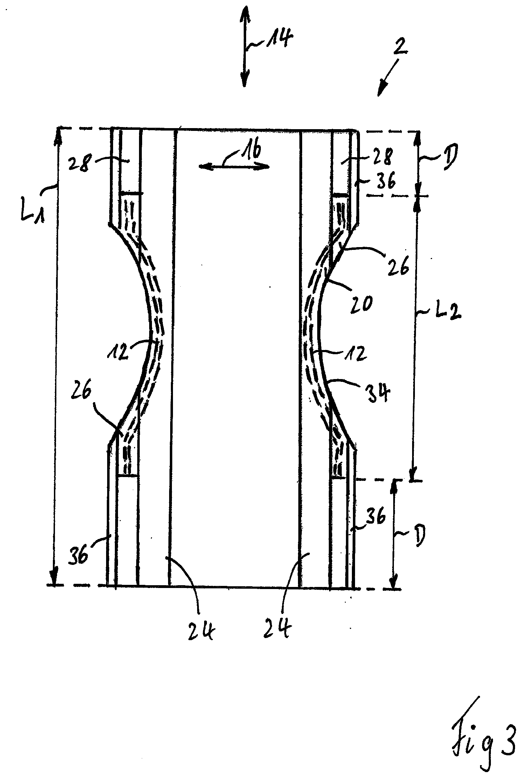

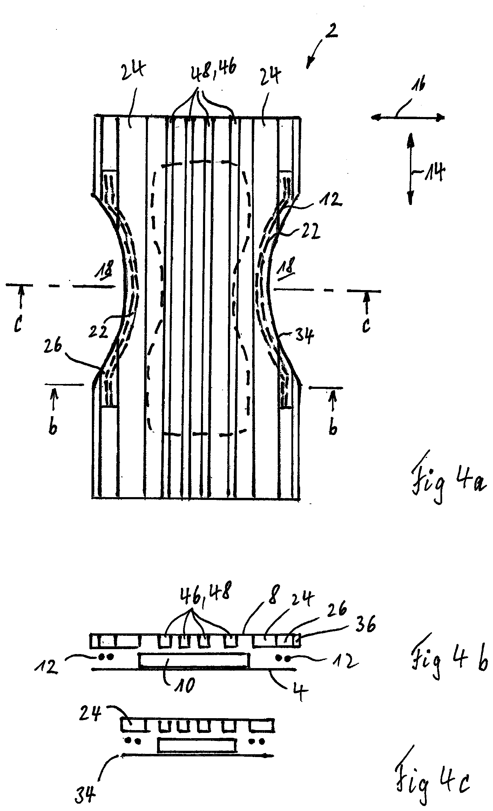

Die Figuren 1 und 2 verdeutlichen das erfindungsgemäße Verfahren zur Herstellung von einen Absorptionskörper umfassenden Schichtstrukturen 2 in endloser Abfolge, die in vereinzelter Form in den Figuren 3 und 4 dargestellt sind. Beispielsweise kann es sich bei diesen Schichtstrukturen 2 auch um an sich fertig verwendbare Hygieneartikel, beispielsweise in Form von Inkontinenzvorlagen oder Inkontinenzeinlagen, handeln odernach Anbringen seitlicher Verschlusslaschen oder seitlicher Abschnitte mit Verschlusslaschen - um offen- und/oder schließbare Windeln. Die wie in den Figuren 1 und 2 dargestellt gefertigten Schichtstrukturen umfassen zumindest die folgenden Komponenten: Einen körperabgewandten Flachmaterialabschnitt 4, insbesondere in Form eines flüssigkeitsundurchlässigen thermoplastischen Folienmaterials, einen körperzugewandten Flachmaterialabschnitt 6 in Form eines Vlieses 8, einen zwischen den Flachmaterialabschnitt 4 und das Vlies 8 geförderten Absorptionskörper 10 sowie beidseitig des Absorptionskörpers erstreckte Eiastifizierungsmittel 12, die wie der Absorptionskörper 10 sandwichartig zwischen dem Flachmaterialabschnitt 4 und dem Vlies 8 aufgenommen und durch noch zu erläuternden Kleberauftrag fixiert sind. Bezugszeichen 13 bezeichnet eine Maschinen- oder Zuführrichtung, die mit einer Produktlängsrichtung 14 fluchtet und orthogonal zu einer Produktquerrichtung 16 ist. Die Elastifizierungsmittel 12 erstrecken sich während des Herstellungsprozesses zwar in der Maschinenrichtung 13, jedoch nicht durchgehend linear, sondern sie erstrecken sich entlang zumindest eines Teils von Beinöffnungen oder Beinausschnitten 18 bogenförmig gekrümmt.

Zur Fixierung der Elastifizierungsmittel 12 in Randbereichen 20 zwischen dem Flachmaterialabschnitt 4 und dem diesbezüglich körperzugewandten Vlies 8 wird flächenhaft aufgebrachter Kleber verwandt. Hierfür wird auf eine körperabgewandte Seite 22 des Vlieses 8 ein streifenförmiger, in der Produktlängsrichtung 14 erstreckter erster Kleberauftrag 24 vollflächig aufgebracht. Der beidseits des Absorptionskörpers 10 im Bereich der intendierten Anordnung der Elastifizierungsmittel 12 aufgebrachte erste Kleberauftrag 24 ist in der Maschinenrichtung 13 vorzugsweise endlos, d.h. kontinuierlich durchgehend auf das Vlies 8 aufgebracht.

In Produktquerrichtung 16 außerhalb des ersten Kleberauftrags 24 wird ein zweiter Kleberauftrag 26 aufgebracht, jedoch nicht in Maschinenrichtung 13 durchgehend, sondern getaktet, d.h. unterbrochen. Dieser zweite Kleberauftrag 26 wird derart getaktet auf das Vlies 8 aufgebracht, dass er in Produktlängsrichtung 14 sowohl nach hinten als auch nach vorn vor dem ersten Kleberauftrag 24 endet, also in der Produktlängsrichtung 14 kürzer ist als dieser. Man ersieht am besten aus Figur 3, dass der zweite Kleberauftrag 26 in Produktlängsrichtung 14 hinten und vorn jeweils um eine Distanz D kürzer ist als der erste Kleberauftrag 24. Dessen Gesamtlänge ist mit L1 bezeichnet. Die Gesamtlänge des zweiten Kleberauftrags 26 ist in Figur 3 mit L2 eingezeichnet. Vorteilhafterweise beträgt die Distanz D vorn und hinten jeweils zwischen 15 % und 30 % der Gesamtlänge L1 des ersten Kleberauftrags 24. Auf diese Weise entsteht bei der Schichtstruktur 2 zwischen dem körperabgewandten Flachmaterialabschnitt 4 und dem Vlies 8 in der Produktlängsrichtung 14 ein kleberfreier Bereich 28 der Länge D. Wenn wie in Figur 2 dargestellt die Elastifizierungsmittel 12 in der Maschinenrichtung 13 bzw. der Produktlängsrichtung 14 durch dieser kleberfreien Bereiche 28 hindurch geführt werden, so sind sie dort nicht mit den chassisbildenden Hüllmaterialien, also dem Flachmaterialabschnitt 4 und dem Vlies 8, fixiert. Dies erweist sich als vorteilhaft, da die Elastifizierungsmittel 12 in diesem kleberfreien Bereich 28 prozesstechnisch verhältnismäßig einfach deelastifiziert werden können, indem sie beispielsweise an nur einer Stelle geschnitten oder in sonstiger Weise ihrer elastifizierenden Wirkung benommen werden können. Da die Elastifizierungsmittel 12 im vorgespannten Zustand zwischen die Hüllmaterialien gefördert und im Leimbett des ersten und zweiten Kleberauftrags 24, 26 laminatartig fixiert worden sind, können sie sich, wenn sie geschnitten werden, in die in den Figuren 2 bis 4 dargestellte Konfiguration zusammenziehen. Eine Schneidstation zum Durchtrennen der Eiastifizierungsmittel 12 und vorzugsweise gleichzeitig zum Vereinzeln der Schichtstrukturen 2 ist in Figur 2 mit Bezugszeichen 30 bezeichnet. Eine Schneidstation zum Konturieren der Beinöffnungen oder Beinausschnitte 18 trägt das Bezugszeichen 32. Man erkennt aus Figur 2, dass vermittels der Schneidstation 32 ein Konturschnift durch den ersten und zweiten Kleberauftrag 24, 26 geführt wird, so dass im beispielhaft dargestellten Fall der erste und der zweite Kleberauftrag 24, 26 randbündig mit Längsrändern 34 des Vlieses 8 im Bereich des Konturschnitts ausgebildet sind.

Auf die körperabgewandte Seite 22 des Vlieses 8 wird bei der beispielhaft dargestellten bevorzugten Ausführungsform der Erfindung beidseits in Produktquerrichtung 16 außerhalb des zweiten Kleberauftrags 26 und außerhalb der beidseitigen Elastifizierungsmittel 12 ein streifenförmiger ebenfalls vollflächig aufgebrachter und in der Produktlängsrichtung 14 erstreckter dritter Kleberauftrag 36 vorgesehen. Der dritte Kleberauftrag 36 verläuft vorzugsweise randbündig mit den geraden Längsrändern 34 des Vlieses vor dem Anbringen der Konturschnitte zur Bildung der Beinöffnungen oder Beinausschnitte 18. Hierdurch werden der Flachmaterialabschnitt 4 und das Vlies 8 in der Produktlängsrichtung 14 kontinuierlich, also unterbrechungsfrei miteinander verbunden. Der dritte Kleberauftrag 36 erfüllt aber eine weitere Funktion. Er begrenzt den bereits erwähnten kleberfreien Bereich 28. Somit ist der jeweilige kleberfreie Bereich 28 in Produktquerrichtung 16 von innen von dem jeweiligen ersten Kleberauftrag 24 und in Produktquerrichtung 16 von außen von dem dritten Kleberauftrag 36 begrenzt. Auf diese Weise ist ein langgestreckter kanalförmiger Abschnitt gebildet, in dem die Elastifizierungsmittel 12 unfixiert und in der Maschinenrichtung 13 zunächst kontinuierlich durchgehend geführt sind. Sie können daher in an sich bekannter Weise abgehoben und geschnitten werden oder im Zuge eines Vereinzelungsschnitts geschnitten werden, ohne dass hierdurch der flüssigkeitsundurchlässige Flachmaterialabschnitt 4 in Mitleidenschaft gezogen wird.

Mit Bezugszeichen 38 ist ein Andrückwalzenpaar im Bereich des ersten bis dritten Kleberauftrags 24, 26, 36 bzw. im Bereich der Elastifizierungsmittel 12 bezeichnet. Bei Bezugszeichen 40 sind Auslenkstifte angedeutet, mittels derer die Eiastifizierungsmittel 12 zur Ausbildung des bogenförmig gekrümmten Verlaufs in Produktquerrichtung 16 hin- und herführbar sind. Mit Bezugszeichen 42 ist in Figur 2 eine Kleberauftragsvorrichtung für den ersten Kleberauftrag 24 und im beispielhaft dargestellten Fall auch für den dritten Kleberauftrag 36 bezeichnet. Für den zweiten Kleberauftrag 26 ist eine weitere Kleberauftragsvorrichtung 44 dargestellt. Bei den Kleberauftragsvorrichtungen handelt es sich um Kontaktbeleimungsvorrichtungen, die eine in der Produktquerrichtung 16 erstreckte schlitzförmige Kleberaustrittsöffnung aufweisen. Die Kleberauftragsvorrichtungen 42 und 44 können wie eingangs erläutert ausgebildet sein.

Figur 4a zeigt eine der Figur 3 entsprechende Schichtstruktur 2, wobei zusätzlich in Produktquerrichtung 16 zwischen dem linken und dem rechten ersten Kleberauftrag 24 ein vierter Kleberauftrag 46 vorgesehen ist, und zwar in Form von mehreren im beispielhaft dargestellten Fall parallel zueinander und in Produktlängsrichtung 14 erstreckten Kleberspuren 48. Diese Kleberspuren 48 sind wiederum streifenförmig und vollflächig aufgebracht, sie haben aber vorteilhafterweise und vorzugsweise eine geringere Breite als der erste Kleberauftrag 24. Sie dienen zur Fixierung des Vlieses 8 mit einer darunter liegenden Schicht, also insbesondere mit dem Absorptionskörper 10 und mit dem körperabgewandten Flachmaterialabschnitt 4 außerhalb des Absorptionskörpers 10. Figuren 4b und 4c zeigen stark schematische nicht maßstabsgetreue Schnittansichten mit Schnittebene b-b bzw. c-c in Fig 4a, die den jeweiligen Kleberauftrag verdeutlichen sollen.

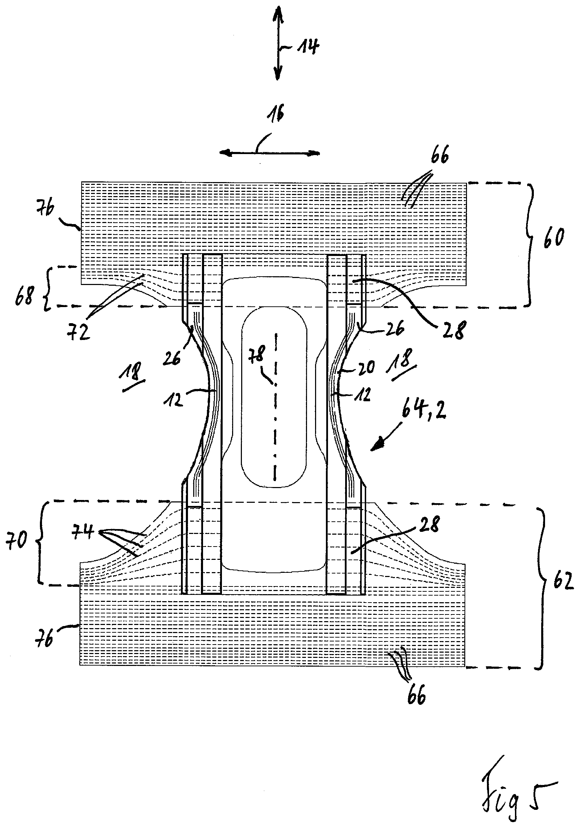

Schließlich zeigt Figur 5 eine Draufsicht auf einen weiteren erfindungsgemäßen Hygieneartikel in Höschenform im eben ausgebreiteten Zustand, also vor der Verbindung seitlicher Längsrandbereiche. Der in Figuren 5 und 6 dargestellte höschenförmige Hygieneartikel ist aus drei Komponenten ausgebildet, nämlich aus einem vorderen Bauchabschnitt 60, einem hinteren Rückenabschnitt 62 und einem diese beiden in Produktlängsrichtung 14 überbrückenden Schrittabschnitt 64. Bei dem Schrittabschnitt 64 handelt es sich im beispielhaft dargestellten Fall um die in den vorhergehenden Figuren beschriebene Schichtstruktur 2. Der Schrittabschnitt 64, d. h. die Schichtstruktur 2, ist in im jeweiligen Überlappungsbereich mit dem Bauchabschnitt 60 und dem Rückenabschnitt 62 mittels Kleber fixiert. Die Art und Weise, Anordnung und Ausbildung der Kleberfixierung ist in DE 102007 055524 A1 beschrieben, und es wird der diesbezügliche Inhalt dieser DE 102007055524 A1 durch Bezugnahme in die vorliegende Anmeldung einbezogen. Diese Art und Weise, Anordnung und Ausbildung der Kleberfixierung ist bevorzugt, aber nicht zwingend. Weiter vorzugsweise kann die Anordnung und Ausbildung der Kleberfixierung wie in DE 102012208393 A1 beschrieben, erfolgen und es wird der diesbezügliche Inhalt dieser DE 102012208393 A1 durch Bezugnahme in die vorliegende Anmeldung einbezogen.

Man erkennt in den Figuren 5 und 6, dass im Bauchabschnitt 60 und im Rückenabschnitt 62 jeweils in Produktquerrichtung 16 bzw. in Hüftumfangsrichtung und parallel zueinander verlaufende Elastifizierungsmittel 66 vorgesehen sind, welche den Bauchabschnitt 60 bzw. den Rückenabschnitt 62 flächenhaft in Produktquerrichtung 16 elastifizieren. In einem den Beinöffnungen 18 zugewandten Bereich 68, 70 von Bauchabschnitt 60 bzw. Rückenabschnitt 62 sind Elastifizierungsmittel 72, 74 vorgesehen, die sich ausgehend von seitlichen Längsnahtbereichen 76 in Richtung auf eine Längsmittelachse 78 fächerförmig erweitern. Ausbildung und Anordnung der in Hüftumfangsrichtung verlaufenden Elastifizierungsmittel 66 und der bogenförmig sich auffächernden Elastifizierungsmittel 72, 74 sind in DE 102007055524 A1 beschrieben, so dass der diesbezügliche Inhalt von DE 102007055524 A1 durch Bezugnahme in die vorliegende Anmeldung einbezogen wird. Dies gilt auch für den Spannungsverlauf in den Elastifizierungsmitteln 72 und 74 und für die in Richtung der Pfeile 80 (Fig. 6) abnehmenden Flächenrückstellkräfte in den den Beinöffnungen 18 zugewandten Bereichen 68, 70 von Bauchabschnitt 60 bzw. Rückenabschnitt 62.

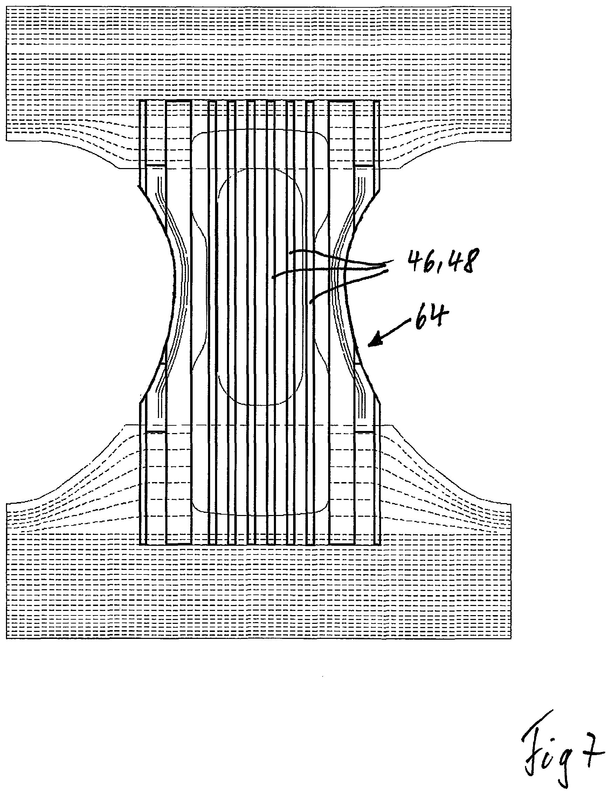

Schließlich zeigt Figur 7 einen höschenförmigen Hygieneartikel entsprechend Figuren 5 und 6, wobei der Schrittabschnitt 64 entsprechend der Schichtstruktur nach Figuren 4a bis c ausgebildet ist, also zusätzlich einen vierten Kleberauftrag 46 mit in Produktlängsrichtung 14 erstreckten Kleberspuren 48 aufweist. Weitere Unterschiede zu dem Hygieneartikel nach Figur 6 bestehen nicht.

Man erkennt bei dem höschenförmigen Hygieneartikel nach Figuren 5 bis 7, dass der jeweilige zweite Kleberauftrag 26 in Produktlängsrichtung 14 ungefähr bei Beginn der Überlappung von Schrittabschnitt 64 und Bauchabschnitt 60 bzw. von Schrittabschnitt 64 und Rückenabschnitt 62 endet. Dies bedeutet, dass die den Beinöffnungen 18 zugeordneten Elastifizierungsmittel 12 ungefähr bis zu diesem Bereich eine Elastifizierung der die Beinöffnungen 18 begrenzenden Randbereiche 20 bewirken. Es erweist sich weiter als vorteilhaft, dass die in der Maschinenrichtung 13 zwar endlos zugeführten und laminierten Elastifizierungsmittel 12 infolge des Schneidvorgangs den kleberfreien Bereich 28 der Schichtstruktur 2 nicht elastifizieren. Dieser kleberfreie Bereich 28 ist bei dem Hygieneartikel gemäß Figuren 5 bis 7 in Überlappung mit dem Bauchabschnitt 60 bzw. Rückenabschnitts 62 angeordnet. Auf diese Weise üben die vor dem Schneidvorgang zwar endlos zugeführten Elastifizierungsmittel 12 keine störende und mit den Elastifizierungsmitteln 66, 72 und 74 des Bauchabschnitts 60 und Rückenabschnitts 64 interferierende Wirkung aus.

## Patentansprüche

1. Hygieneartikel, wie insbesondere Windel, Höschenwindel, Inkontinenzvorlage, mit einer Produktlängsrichtung (14) und einer Produktquerrichtung (16), mit einem Absorptionskörper (10) und mit Beinöffnungen oder Beinausschnitte (18) begrenzenden beidseitigen Randbereichen (20), wobei in Produktquerrichtung (16) beidseits außerhalb des Absorptionskörpers (10) und entlang zumindest eines Teils der beidseitigen Randbereiche (20) langgestreckte Elastifizierungsmittel (12) vorgesehen und zwischen Flachmaterialabschnitten (4, 6) des Hygieneartikels fixiert sind, so dass eine Elastifizierung entlang zumindest eines Teils der Beinöffnungen oder der Beinausschnitte (18) resultiert, wobei die Elastifizierungsmittel (12) entlang zumindest eines Teils der Beinöffnungen oder der Beinausschnitte (18) bogenförmig gekrümmt verlaufen, wobei ein körperzugewandter Flachmaterialabschnitt (6) ein Vlies (8) umfasst, **dadurch gekennzeichnet, dass** das Vlies (8) im Bereich der beidseitigen Elastifizierungsmittel (12) und zu deren Fixierung auf seiner körperabgewandten Seite (22) einen streifenförmigen, vollflächig aufgebrachten und in der Produktlängsrichtung (14) erstreckten ersten Kleberauftrag (24) aufweist und dass das Vlies (8) im Bereich der beidseitigen Elastifizierungsmittel (12) und zu deren Fixierung auf seiner körperabgewandten Seite (22) beidseits in Produktquerrichtung (16) außerhalb des ersten Kleberauftrags (24) einen streifenförmigen, vollflächig aufgebrachten und in der Produktlängsrichtung (14) erstreckten zweiten Kleberauftrag (26) aufweist, der in Produktquerrichtung (16) mit dem ersten Kleberauftrag (24) teilweise überlappt oder unmittelbar an den ersten Kleberauftrag (24) angrenzt oder von dem ersten Kleberauftrag (24) in Produktquerrichtung (16) nach außerhalb beabstandet ist, und dass der zweite Kleberauftrag (26) in Produktlängsrichtung (14) nach vorn und in Produktlängsrichtung (14) nach hinten jeweils kürzer ist als der erste Kleberauftrag (24), also vor diesem endet, und infolge seitlicher Konturierung der beidseitigen Randbereiche (20) zur Bildung der Beinöffnungen oder Beinausschnitte (18) auch unterbrochen oder konturiert sein kann, und dass die langgestreckten Elastifizierungsmittel (12) durch den ersten und den zweiten Kleberauftrag (24, 26) zwischen dem Vlies (8) und einem gegenüber dem Vlies (8) körperabgewandten Flachmaterialabschnitt (4) fixiert sind.

2. Hygieneartikel nach Anspruch 1, **dadurch gekennzeichnet, dass** der bogenförmig gekrümmte Verlauf der beidseitigen Elastifizierungsmittel (12) mindestens durch den ersten Kleberauftrag (24) verläuft und fixiert ist.

3. Hygieneartikel nach Anspruch 1 oder 2, **dadurch gekennzeichnet, dass** ein an den bogenförmigen Verlauf der Elastifizierungsmittel (12) anschließender linearer Verlauf der Elastifizierungsmittel nur durch den zweiten Kleberauftrag verläuft und fixiert ist.

4. Hygieneartikel nach Anspruch 1, 2 oder 3, **dadurch gekennzeichnet, dass** der erste Kleberauftrag (24) in Produktquerrichtung (16) eine Breite von wenigstens 20 mm, insbesondere von wenigstens 25 mm, und von höchstens 40 mm, insbesondere von höchstens 35 mm aufweist.

5. Hygieneartikel nach einem oder mehreren der vorstehenden Ansprüche, **dadurch gekennzeichnet, dass** der erste und/oder der zweite Kleberauftrag (24, 26) ein Flächengewicht von wenigstens 1 g/m², insbesondere von wenigstens 2 g/m², insbesondere von höchstens 20 g/m², insbesondere von höchstens 10 g/m², insbesondere von höchstens 7 g/m² und weitere insbesondere von 2-5 g/m² aufweist.

6. Hygieneartikel nach einem oder mehreren der vorstehenden Ansprüche, **dadurch gekennzeichnet, dass** der zweite Kleberauftrag (26) in Produktquerrichtung (16) eine Breite von wenigstens 5 mm, insbesondere von wenigstens 10 mm, insbesondere von wenigstens 15 mm und von höchstens 30 mm, insbesondere von höchstens 25 mm, und weiter insbesondere von höchstens 20 mm aufweist.

7. Hygieneartikel nach einem oder mehreren der vorstehenden Ansprüche, **dadurch gekennzeichnet, dass** der zweite Kleberauftrag (26) in Produktlängsrichtung (14) nach vorn und in Produktlängsrichtung (14) nach hinten jeweils um eine Distanz (D) kürzer ist, die wenigstens 15 %, insbesondere wenigstens 20 % und höchstens 35 %, insbesondere höchstens 30 % der Gesamtlänge (L₁) des ersten Kleberauftrags (24) in Produktlängsrichtung (14) beträgt.

8. Hygieneartikel nach einem oder mehreren der vorstehenden Ansprüche, **dadurch gekennzeichnet, dass** das Vlies (8) auf seiner körperabgewandten Seite (22) und beidseits in Produktquerrichtung (16) außerhalb des zweiten Kleberauftrags (26) und außerhalb der beidseitigen Elastifizierungsmittel (12) einen streifenförmigen, vollflächig aufgebrachten und in der Produktlängsrichtung (14) erstreckten dritten Kleberauftrag (36) aufweist, wobei insbesondere in Produktquerrichtung (16) zwischen dem ersten und dem dritten Kleberauftrag (24, 36) und in Produktlängsrichtung (14) hinten und vorn ein kanalförmiger kleberfreier Abschnitt (28) zwischen dem Vlies (8) und dem dazu körperabgewandt angeordneten Flachmaterialabschnitt (4) gebildet ist.

9. Hygieneartikel nach Anspruch 8, **dadurch gekennzeichnet, dass** der dritte Kleberauftrag (36) zumindest abschnittsweise randbündig mit Längsrändern (34) des Vlieses (8) ausgebildet ist.

10. Hygieneartikel nach einem oder mehreren der vorstehenden Ansprüche, **dadurch gekennzeichnet, dass** der zweite Kleberauftrag (26), und insbesondere zusätzlich der erste Kleberauftrag (24) durch die seitliche Konturierung der beidseitigen Randbereiche (20) zur Bildung von Beinöffnungen oder Beinausschnitten (18) abschnittsweise randbündig mit Längsrändern (34) des Vlieses (8) ausgebildet sind.

11. Hygieneartikel nach einem oder mehreren der vorstehenden Ansprüche, **dadurch gekennzeichnet, dass** sich der erste und der zweite Kleberauftrag (24, 26) durch unterschiedliche Klebermaterialien und/oder durch unterschiedliches Flächengewicht voneinander unterscheiden.

12. Hygieneartikel nach einem oder mehreren der vorstehenden Ansprüche, **dadurch gekennzeichnet, dass** das Vlies (8) ein Spunbondmaterial, ein Laminat aus Spunbondlagen (S) und Meltblownlagen (M) oder ein Stapelfaservliesmaterial oder Kombinationen davon umfasst und insbesondere ein Flächengewicht von mindestens 6 g/m², insbesondere von mindestens 10 g/m², insbesondere von höchstens 30 g/m², insbesondere von höchstens 20 g/m² aufweist.

13. Hygieneartikel nach einem oder mehreren der vorstehenden Ansprüche, **dadurch gekennzeichnet, dass** der körperabgewandte Flachmaterialabschnitt (4) eine thermoplastische Folien- oder Filmschicht, eine insbesondere mikroporöse Folie, oder ein Vlies-Folien-Laminat umfasst.

14. Hygieneartikel nach einem oder mehreren der vorstehenden Ansprüche, **dadurch gekennzeichnet, dass** zur Fixierung der Elastifizierungsmittel (12) nur der erste und der zweite Kleberauftrag (24, 26) verwendet ist.

15. Hygieneartikel nach einem oder mehreren der vorstehenden Ansprüche, **dadurch gekennzeichnet, dass** sich der erste und/oder der dritte (24, 36) in Produktlängsrichtung (14) bis zu Längsenden des Hygieneartikels erstrecken.

16. Hygieneartikel nach einem oder mehreren der vorstehenden Ansprüche, **gekennzeichnet durch** eine den Absorptionskörper (10) umfassende Schichtstruktur (2), welche die beidseitigen Randbereiche (20) bildet, in denen die langgestreckten Elastifizierungsmittel (12) entlang zumindest eines Teils der Beinöffnungen oder Beinausschnitte (18) vorgesehen sind.

17. Hygieneartikel nach Anspruch 16, mit einem vorderen Bauchabschnitt (60) und einem hinteren Rückenabschnitt (62), die von separaten und in Produktlängsrichtung (14) voneinander beabstandeten Komponenten gebildet sind, jedoch zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen (76) herstellerseitig miteinander verbunden sind, und mit einem den Absorptionskörper (10) aufweisenden Schrittabschnitt (64), der sich in der Produktlängsrichtung (14) zwischen Bauchabschnitt (60) und Rückenabschnitt (62) erstreckt und diese überbrückt und an den Bauchabschnitt (60) und an den Rückenabschnitt (62) in einem jeweiligen Überlappungsbereich unlösbar angefügt ist, wobei der Bauchabschnitt (60), der Rückenabschnitt (62) und der Schrittabschnitt (64) gemeinsam die Beinöffnungen (18) begrenzen, wobei in dem Bauchabschnitt (60) und in dem Rückenabschnitt (62) in Produktquerrichtung (16) verlaufende weitere Elastifizierungsmittel (66) vorgesehen sind, die den Bauchabschnitt (60) und den Rückenabschnitt (62) flächenhaft elastifizieren, und wobei der Schrittabschnitt (64) die den Absorptionskörper (10) umfassende Schichtstruktur (2) bildet, wobei insbesondere sich der erste und/oder der dritte Kleberauftrag (24, 36) in Produktlängsrichtung (14) bis zu Längsenden der den Absorptionskörper (10) umfassenden Schichtstruktur (2) erstrecken.

18. Verfahren zum Herstellen eines Hygieneartikels oder einer einen Absorptionskörper (10) umfassenden Schichtstruktur (2) eines Hygieneartikels nach einem oder mehreren der vorstehenden Ansprüche, wobei die langgestreckten Elastifizierungsmittel (12) entlang zumindest eines Teils der die beidseitigen Beinöffnungen oder Beinausschnitte (18) begrenzenden Randbereiche (20) zwischen Flachmaterialabschnitten (4, 6) des Hygieneartikels fixiert werden, die folgenden Merkmale umfassend:
- Zuführen eines den körperzugewandten Flachmaterialabschnitt (6) bildenden endlosen Vlieses (8) in einer Maschinenrichtung (13), die der späteren Produktlängsrichtung (14) entspricht,
- kontinuierliches vollflächiges Aufbringen des ersten Kleberauftrags (24) auf die körperabgewandte Seite (22) des Vlieses (8) in den beidseitigen Randbereichen (20) in der Maschinenrichtung (13),
- getaktetes vollflächiges Aufbringen des zweiten Kleberauftrags (26) in der Maschinenrichtung (13) auf die körperabgewandte Seite des Vlieses (8) in den beidseitigen Randbereichen (20) in Produktquerrichtung (16) außerhalb des ersten Kleberauftrags (24), derart, dass der zweite Kleberauftrag (26) in Produktquerrichtung (16) mit dem ersten Kleberauftrag (24) teilweise überlappt oder unmittelbar an den ersten Kleberauftrag (24) angrenzt oder von dem ersten Kleberauftrag (24) in Produktquerrichtung (16) beabstandet ist, wobei der zweite Kleberauftrag (26) derart getaktet aufgebracht wird, dass er in Produktlängsrichtung (14) nach vorn und hinten kürzer ist als der erste Kleberauftrag (24), also vor diesem endet,
- Zuführen einer den körperabgewandten Flachmaterialabschnitt (4) bildenden endlosen Flachmaterialbahn in der Maschinenrichtung (13),
- Zuführen der beidseitigen Elastifizierungsmittel (12) in der Maschinenrichtung (13) zwischen das Vlies (8) urid die körperabgewandte Flachmaterialbahn zumindest abschnittsweise im Bereich des ersten und des zweiten Kleberauftrags (24, 26),
- Zusammenbringen des endlosen Vlieses (8) und der körperabgewandten Flachmaterialbahn unter Zwischenanordnung der Elastifizierungsmittel (12) und Fixieren der Elastifizierungsmittel (12) durch den ersten und zweiten Kleberauftrag (24, 26),
- Deelastifizieren der Elastifizierungsmittel (12) in einem im Zuge der Taktung des zweiten Kleberauftrags (26) in Produktlängsrichtung (14) kleberfrei verbliebenen Bereich (28).

19. Verfahren nach Anspruch 18, weiter **gekennzeichnet durch** vollflächiges Aufbringen eines dritten Kleberauftrags (36) in der Maschinenrichtung (13) auf die körperabgewandte Seite (22) des Vlieses (8) in den beidseitigen Randbereichen (20) in Produktquerrichtung (16) außerhalb des zweiten Kleberauftrags (26) und außerhalb der beidseitigen Elastifizierungsmittel (12), insbesondere dass der dritte Kleberauftrag (36) in der Maschinenrichtung (13) kontinuierlich aufgebracht werden.

20. Verfahren nach einem oder mehreren der Ansprüche 18-19, **dadurch gekennzeichnet, dass** zur Fixierung der Elastifizierungsmittel (12) nur der erste und der zweite Kleberauftrag (24, 26) verwendet werden und die körperabgewandte Flachmaterialbahn unbeschichtet bleibt in diesem Bereich.

21. Verfahren nach einem oder mehreren der Ansprüche 18-20, **dadurch gekennzeichnet, dass** zur Aufbringung des ersten und des zweiten Kleberauftrags (24, 26), insbesondere auch zur Aufbringung des dritten Kleberauftrags (36), eine Kleberauftragsvorrichtung (42, 44) verwendet wird, welche im nächsten Kontakt zum Vlies aus insbesondere schlitzförmigen Kleberaustrittsöffnungen Klebermaterial abgibt.

22. Verfahren nach einem oder mehreren der Ansprüche 18-21, **dadurch gekennzeichnet, dass** nach Zusammenbringen des Vlieses (8) und der körperabgewandten Flachmaterialbahn beidseits in der Maschinenrichtung (13) geführte Konturschnitte angebracht werden, um die Beinöffnungen oder die Beinausschnitte (18) zu bilden.

23. Verfahren nach einem oder mehreren der vorstehenden Ansprüche 18-22, **dadurch gekennzeichnet, dass** hiermit eine endlose Bahn von jeweils einen Absorptionskörper (10) umfassenden Schichtstrukturen (2) gefertigt wird, die die körperabgewandte Flachmaterialbahn und das körperzugewandte Vlies (8) und eine endlose Folge von in Maschinenrichtung (13) aufeinander geförderten Absorptionskörpern (10) umfasst, die zwischen die körperabgewandte Flachmaterialbahn und das körperzugewandte Vlies (8) gefördert und angeordnet werden, und dass quer zur Maschinenrichtung (13) ein Vereinzelungsschnitt zur Bildung der Schichtstrukturen (2) ausgeführt wird, dass die vereinzelten Schichtstrukturen (2) um 90° gedreht und in überbrückende Anordnung mit einer Bauchabschnittbahn (60) und einer Rückenabschnittbahn (62) gebracht werden und im Überlappungsbereich mit der Bauchabschnittbahn (60) und der Rückenabschnittbahn (64) unlösbar fixiert werden

## Claims

1. A sanitary article, such as, in particular, a diaper, a pants-type diaper, an incontinence pad, having a product longitudinal direction (14) and a product transverse direction (16), having an absorption body (10) and having bilateral marginal regions (20) which delimit leg openings or leg cutouts (18), wherein, on both sides outside of the absorption body (10) in the product transverse direction (16), and along at least a part of the bilateral marginal regions (20), elongated elasticating means (12) are provided and fixed between flat material portions (4, 6) of the sanitary article, so that an elastication along at least a part of the leg openings or leg cutouts (18) results, wherein the elasticating means (12) run in an arcuately curved path along at least a part of the leg openings or leg cutouts (18), and wherein a body-facing flat material portion (6) comprises a nonwoven fabric (8), **characterized in that** the nonwoven fabric (8), in the region of the bilateral elasticating means (12) and for the fixing thereof, has on its body-remote side (22) a strip-shaped first adhesive coating (24), which is applied over the whole surface and is extended in the product longitudinal direction (14), and **in that** the nonwoven fabric (8), in the region of the bilateral elasticating means (12) and for the fixing thereof, has on its body-remote side (22), on both sides outside of the first adhesive coating (24) in the product transverse direction (16), a strip-shaped second adhesive coating (26), which is applied over the whole surface and is extended in the product longitudinal direction (14) and which in the product transverse direction (16) partially overlaps with the first adhesive coating (24) or directly adjoins the first adhesive coating (24) or is outwardly distanced from the first adhesive coating (24) in the product transverse direction (16), and **in that** the second adhesive coating (26), to the fore in the product longitudinal direction (14) and to the rear in the product longitudinal direction (14), is respectively shorter than the first adhesive coating (24), i.e. ends before the latter, and as a consequence of lateral contouring of the bilateral marginal regions (20) for the formation of the leg openings or leg cutouts (18) can also be interrupted or contoured, and **in that** the elongated elasticating means (12) are fixed by the first and the second adhesive coating (24, 26) between the nonwoven fabric (8) and a, in relation to the nonwoven fabric (8), body-remote flat material portion (4).

2. The sanitary article as claimed in claim 1, **characterized in that** the arcuately curved path of the bilateral elasticating means (12) runs at least through the first adhesive coating (24) and is fixed.

3. The sanitary article as claimed in claim 1 or 2, **characterized in that** a linear path of the elasticating means, which adjoins the arcuate path of the elasticating means (12), runs only through the second adhesive coating and is fixed.

4. The sanitary article as claimed in claim 1, 2 or 3, **characterized in that** the first adhesive coating (24) has in the product transverse direction (16) a width of at least 20 mm, in particular of at least 25 mm, and of no more than 40 mm, in particular of no more than 35 mm.

5. The sanitary article as claimed in one or more of the preceding claims, **characterized in that** the first and/or the second adhesive coating (24, 26) has a weight per unit area of at least 1 g/m², in particular of at least 2 g/m², in particular of no more than 20 g/m², in particular of no more than 10 g/m², in particular of no more than 7 g/m², and further particularly of 2 - 5 g/m².

6. The sanitary article as claimed in one or more of the preceding claims, **characterized in that** the second adhesive coating (26) has in the product transverse direction (16) a width of at least 5 mm, in particular of at least 10 mm, in particular of at least 15 mm and of no more than 30 mm, in particular of no more than 25 mm, and further particularly of no more than 20 mm.

7. The sanitary article as claimed in one or more of the preceding claims, **characterized in that** the second adhesive coating (26), to the fore in the product longitudinal direction (14) and to the rear in the product longitudinal direction (14), is respectively shorter by a distance (D) which amounts to at least 15 %, in particular at least 20 % and no more than 35 %, in particular no more than 30 %, of the total length (L₁) of the first adhesive coating (24) in the product longitudinal direction (14).

8. The sanitary article as claimed in one or more of the preceding claims, **characterized in that** the nonwoven fabric (8) has on its body-remote side (22), and on both sides outside of the second adhesive coating (26) and outside of the bilateral elasticating means (12) in the product transverse direction (16), a strip-shaped third adhesive coating (36) applied over the whole surface and extended in the product longitudinal direction (14), wherein in particular in the product transverse direction (16) between the first and the third adhesive coating (24, 36) and at the rear and front in the product longitudinal direction (14), a channel-shaped adhesive-free portion (28) is formed between the nonwoven fabric (8) and the flat material portion (4) arranged, for this purpose, facing away from the body.

9. The sanitary article as claimed in claim 8, **characterized in that** the third adhesive coating (36), at least in some sections, is configured marginally flush with longitudinal margins (34) of the nonwoven fabric (8).

10. The sanitary article as claimed in one or more of the preceding claims, **characterized in that** the second adhesive coating (26), and in particular additionally the first adhesive coating (24), through the lateral contouring of the bilateral marginal regions (20) to form leg openings or leg cutouts (18), are in some sections configured marginally flush with longitudinal margins (34) of the nonwoven fabric (8).

11. The sanitary article as claimed in one or more of the preceding claims, **characterized in that** the first and the second adhesive coating (24, 26) differ from each other by way of different adhesive materials and/or different weight per unit area.

12. The sanitary article as claimed in one or more of the preceding claims, **characterized in that** the nonwoven fabric (8) comprises a spunbonded material, a laminate of spunbonded layers (S) and meltblown layers (M) or a staple fiber nonwoven material, or combinations thereof, and has in particular a weight per unit area of at least 6 g/m², in particular of at least 10 g/m², in particular of no more than 30 g/m², in particular of no more than 20 g/m².

13. The sanitary article as claimed in one or more of the preceding claims, **characterized in that** the body-remote flat material portion (4) comprises a thermoplastic foil or film layer, an in particular microporous foil, or a nonwoven fabric-foil laminate.

14. The sanitary article as claimed in one or more of the preceding claims, **characterized in that**, for the fixing of the elasticating means (12), only the first and the second adhesive coating (24, 26) is used.

15. The sanitary article as claimed in one or more of the preceding claims, **characterized in that** the first and/or the third adhesive coating (24, 36) extend in the product longitudinal direction (14) up to longitudinal ends of the sanitary article.

16. The sanitary article as claimed in one or more of the preceding claims, **characterized by** a layered structure (2) comprising the absorption body (10) and forming the bilateral marginal regions (20), in which the elongated elasticating means (12) are provided along at least a part of the leg openings or leg cutouts (18).

17. The sanitary article as claimed in claim 16, having a front abdominal portion (60) and a rear spinal portion (62), which are formed by separate components that are spaced apart in the product longitudinal direction (14), yet, for the formation of an abdominal and spinal band passing in the transverse direction or hip circumferential direction, are factory-connected to each other at bilateral side seam regions (76) with a hip opening that is closed in the hip circumferential direction, and having a crotch portion (64), which comprises the absorption body (10) and extends in the product longitudinal direction (14) between the abdominal portion (60) and the spinal portion (62) and bridges these and is non-detachably joined to the abdominal portion (60) and to the spinal portion (62) in a respective overlap region, wherein the abdominal portion (60), the spinal portion (62) and the crotch portion (64) jointly delimit the leg openings (18), wherein in the abdominal portion (60) and in the spinal portion (62) are provided further elasticating means (66), which run in the product transverse direction (16) and areally elasticate the abdominal portion (60) and the spinal portion (62), and wherein the crotch portion (64) forms the layered structure (2) comprising the absorption body (10), wherein in particular the first and/or the third adhesive coating (24, 36) extend in the product longitudinal direction (14) up to longitudinal ends of the layered structure (2) comprising the absorption body (10).

18. A method for producing a sanitary article or a layered structure (2), comprising an absorption body (10), of a sanitary article as claimed in one or more of the preceding claims, wherein the elongated elasticating means (12), along at least a part of the marginal regions (20) delimiting the bilateral leg openings or leg cutouts (18), are fixed between flat material portions (4, 6) of the sanitary article, comprising the following features:
- feeding of an endless nonwoven fabric (8), forming the body-facing flat material portion (6), in a machine direction (13) which corresponds to the later product longitudinal direction (14),
- continuous all-over application of the first adhesive coating (24), in the machine direction (13), to the body-remote side (22) of the nonwoven fabric (8) in the bilateral marginal regions (20),
- clocked all-over application of the second adhesive coating (26), in the machine direction (13), to the body-remote side of the nonwoven fabric (8) in the bilateral marginal regions (20) outside of the first adhesive coating (24) in the product transverse direction (16), such that the second adhesive coating (26), in the product transverse direction (16), partially overlaps with the first adhesive coating (24) or directly adjoins the first adhesive coating (24), or is distanced from the first adhesive coating (24) in the product transverse direction (16), wherein the second adhesive coating (26) is applied in a clocked manner such that, to the front and rear in the product longitudinal direction (14), it is shorter than the first adhesive coating (24), i.e. ends before the latter,
- feeding of an endless flat material web, forming the body-remote flat material portion (4), in the machine direction (13),
- feeding of the bilateral elasticating means (12) in the machine direction (13) between the nonwoven fabric (8) and the body-remote flat material web, at least in some sections, in the region of the first and the second adhesive coating (24, 26),
- bringing together of the endless nonwoven fabric (8) and the body-remote flat material web, with the interposition of the elasticating means (12), and fixing of the elasticating means (12) by the first and second adhesive coating (24, 26),
- deelastication of the elasticating means (12), in a region (28) which has remained free of adhesive in the course of the clocking of the second adhesive coating (26), in the product longitudinal direction (14).

19. The method as claimed in claim 18, further **characterized by** all-over application of a third adhesive coating (36), in the machine direction (13), to the body-remote side (22) of the nonwoven fabric (8) in the bilateral marginal regions (20) outside of the second adhesive coating (26) and outside of the bilateral elasticating means (12) in the product transverse direction (16), in particular that the third adhesive coating (36) is applied continuously in the machine direction (13).

20. The method as claimed in one or more of claims 18 - 19, **characterized in that**, for the fixing of the elasticating means (12), only the first and the second adhesive coating (24, 26) are used and the body-remote flat material web remains uncoated in this region.

21. The method as claimed in one or more of claims 18 - 20, **characterized in that**, for the application of the first and the second adhesive coating (24, 26), in particular also for the application of the third adhesive coating (36), an adhesive coating apparatus (42, 44), which in closest contact to the nonwoven fabric delivers adhesive material from, in particular, slit-shaped adhesive discharge openings, is used.

22. The method as claimed in one or more of claims 18 - 21, **characterized in that**, after the nonwoven fabric (8) and the body-remote flat material web have been brought together, contour cuts, conducted in the machine direction (13), are made on both sides in order to form the leg openings or the leg cutouts (18).

23. The method as claimed in one or more of the preceding claims 18-22, **characterized in that** an endless web of layered structures (2), respectively comprising an absorption body (10), is herewith produced, which endless web comprises the body-remote flat material web and the body-facing nonwoven fabric (8) and an endless succession of absorption bodies (10), fed one after another in the machine direction (13), which are conveyed and arranged between the body-remote flat material web and the body-facing nonwoven fabric (8), and **in that**, transversely to the machine direction (13), a separation cut for the formation of the layered structures (2) is performed, **in that** the separated layered structures (2) are rotated through 90° and are brought into bridging arrangement with an abdominal portion web (60) and a spinal portion web (62) and are non-detachably fixed in the overlap region with the abdominal portion web (60) and the spinal portion web (64).

## Revendications

1. Article hygiénique, tel que, en particulier, couche, couche-culotte, protection pour l'incontinence, ayant une direction longitudinale de produit (14) et une direction transversale de produit (16), comprenant un corps absorbant (10) et des zones marginales (20) des deux côtés qui limitent des ouvertures de jambe ou découpes de jambe (18), dans lequel des moyens d'élastification (12) allongés sont prévus dans la direction transversale de produit (16) de part et d'autre à l'extérieur du corps absorbant (10) et le long d'au moins une partie des zones marginales (20) des deux côtés et sont fixés entre des portions de matériau plat (4, 6) de l'article hygiénique de sorte qu'il en résulte une élastification le long d'au moins une partie des ouvertures de jambe ou des découpes de jambe (18), dans lequel lesdits moyens d'élastification (12) s'étendent de manière courbée en arc le long d'au moins une partie des ouvertures de jambe ou des découpes de jambe (18), dans lequel une portion de matériau plat (6) qui est tournée vers le corps comprend un non-tissé (8), **caractérisé par le fait que** le non-tissé (8) comprend un premier enduit de colle (24) en forme de bande appliqué sur toute la surface et s'étendant dans la direction longitudinale de produit (14), au niveau des moyens d'élastification (12) des deux côtés et dans le but de leur fixation sur sa face (22) opposée au corps, et que le non-tissé (8) comprend un deuxième enduit de colle (26) en forme de bande appliqué sur toute la surface et s'étendant dans la direction longitudinale de produit (14), au niveau des moyens d'élastification (12) des deux côtés et dans le but de leur fixation sur sa face (22) opposée au corps, de part et d'autre dans la direction transversale de produit (16), à l'extérieur du premier enduit de colle (24), deuxième enduit de colle qui recouvre en partie ledit premier enduit de colle (24) dans la direction transversale de produit (16) ou est immédiatement contigu au premier enduit de colle (24) ou est espacé du premier enduit de colle (24) vers l'extérieur dans la direction transversale de produit (16), et que, dans la direction longitudinale de produit (14) vers l'avant et dans la direction longitudinale de produit (14) vers l'arrière, ledit deuxième enduit de colle (26) est respectivement plus court que le premier enduit de colle (24), donc se termine en amont de celui-ci, et peut être également interrompu ou contouré par suite de contourage latéral des zones marginales (20) des deux côtés pour la formation des ouvertures de jambe ou découpes de jambe (18), et que lesdits moyens d'élastification (12) allongés sont fixés par les premier et deuxième enduits de colle (24, 26) entre le non-tissé (8) et une portion de matériau plat (4) opposée au corps par rapport au non-tissé (8).

2. Article hygiénique selon la revendication 1, **caractérisé par le fait que** le tracé courbé en arc des moyens d'élastification (12) des deux côtés s'étend au moins à travers le premier enduit de colle (24) et est fixé par celui-ci.

3. Article hygiénique selon la revendication 1 ou 2, **caractérisé par le fait qu'**un tracé linéaire des moyens d'élastification qui est contigu au tracé en arc des moyens d'élastification (12) ne s'étend qu'à travers le deuxième enduit de colle et est fixé par celui-ci.

4. Article hygiénique selon la revendication 1, 2 ou 3, **caractérisé par le fait que**, dans la direction transversale de produit (16), le premier enduit de colle (24) présente une largeur d'au moins 20 mm, en particulier d'au moins 25 mm, et de 40 mm tout au plus, en particulier de 35 mm tout au plus.

5. Article hygiénique selon l'une ou plusieurs des revendications précédentes, **caractérisé par le fait que** le premier et/ou le deuxième enduit(s) de colle (24, 26) présente(nt) un grammage d'au moins 1 g/m², en particulier d'au moins 2 g/m², en particulier de 20 g/m² tout au plus, en particulier de 10 g/m² tout au plus, en particulier de 7 g/m² tout au plus et, encore en particulier, compris entre 2 et 5 g/m².

6. Article hygiénique selon l'une ou plusieurs des revendications précédentes, **caractérisé par le fait que**, dans la direction transversale de produit (16), le deuxième enduit de colle (26) présente une largeur d'au moins 5 mm, en particulier d'au moins 10 mm, en particulier d'au moins 15 mm et de 30 mm tout au plus, en particulier de 25 mm tout au plus, et encore en particulier de 20 mm tout au plus.

7. Article hygiénique selon l'une ou plusieurs des revendications précédentes, **caractérisé par le fait que**, dans la direction longitudinale de produit (14) vers l'avant et dans la direction longitudinale de produit (14) vers l'arrière, ledit deuxième enduit de colle (26) est respectivement plus court d'une distance (D) qui fait au moins 15 %, en particulier au moins 20 % et 35 % tout au plus, en particulier 30 % tout au plus de la longueur totale (L₁) du premier enduit de colle (24) dans la direction longitudinale de produit (14).

8. Article hygiénique selon l'une ou plusieurs des revendications précédentes, **caractérisé par le fait que** le non-tissé (8) comprend un troisième enduit de colle (36) en forme de bande appliqué sur toute la surface et s'étendant dans la direction longitudinale de produit (14), sur sa face (22) opposée au corps et de part et d'autre dans la direction transversale de produit (16) à l'extérieur du deuxième enduit de colle (26) et à l'extérieur des moyens d'élastification (12) des deux côtés, dans lequel, en particulier dans la direction transversale de produit (16) entre les premier et troisième enduits de colle (24, 36) et dans la direction longitudinale de produit (14) à l'arrière et à l'avant, une portion exempte de colle (28) en forme de canal est formée entre le non-tissé (8) et la portion de matériau plat (4) opposée au corps par rapport à cela.

9. Article hygiénique selon la revendication 8, **caractérisé par le fait que** ledit troisième enduit de colle (36) est réalisé de manière à être aligné au bord, au moins par sections, avec des bords longitudinaux (34) du non-tissé (8).

10. Article hygiénique selon l'une ou plusieurs des revendications précédentes, **caractérisé par le fait que**, par le contourage latéral des zones marginales (20) des deux côtés pour la formation d'ouvertures de jambe ou de découpes de jambe (18), le deuxième enduit de colle (26), et en particulier en plus le premier enduit de colle (24), sont réalisés de manière à être alignés au bord, au moins par sections, avec des bords longitudinaux (34) du non-tissé (8).

11. Article hygiénique selon l'une ou plusieurs des revendications précédentes, **caractérisé par le fait que** les premier et deuxième enduits de colle (24, 26) se distinguent l'un de l'autre par des matières adhésives différentes et/ou par un grammage différent.

12. Article hygiénique selon l'une ou plusieurs des revendications précédentes, **caractérisé par le fait que** le non-tissé (8) comprend une matière spunbond, un ensemble laminé en couches spunbond (S) et en couches meltblow (M) ou une matière de non-tissé en fibres discontinues ou des combinaisons de ceux-ci et, en particulier, présente un grammage d'au moins 6 g/m², en particulier d'au moins 10 g/m², en particulier de 30 g/m² tout au plus, en particulier de 20 g/m² tout au plus.

13. Article hygiénique selon l'une ou plusieurs des revendications précédentes, **caractérisé par le fait que** la portion de matériau plat (4) opposée au corps comprend une couche en feuille ou en film thermoplastique, une feuille en particulier microporeuse, ou un ensemble laminé de non-tissé et feuille.

14. Article hygiénique selon l'une ou plusieurs des revendications précédentes, **caractérisé par le fait que**, pour fixer les moyens d'élastification (12), on n'utilise que les premier et deuxième enduits de colle (24, 26).

15. Article hygiénique selon l'une ou plusieurs des revendications précédentes, **caractérisé par le fait que** le premier et/ou le troisième (24, 36) s'étendent dans la direction longitudinale de produit (14) jusqu'à des extrémités longitudinales de l'article hygiénique.

16. Article hygiénique selon l'une ou plusieurs des revendications précédentes, **caractérisé par** une structure stratifiée (2) comprenant le corps absorbant (10), qui forme lesdites zones marginales (20) des deux côtés dans lesquelles les moyens d'élastification (12) allongés sont prévus le long d'au moins une partie des ouvertures de jambe ou découpes de jambe (18).

17. Article hygiénique selon la revendication 16, comprenant une portion ventrale avant (60) et une portion dorsale arrière (62) qui sont formées par des composants séparés et espacés l'un de l'autre dans la direction longitudinale de produit (14), mais qui, pour la formation d'une bande ventrale et dorsale continue dans le sens transversal ou dans le sens du tour de hanches, avec une ouverture de hanche fermée dans le sens du tour de hanches, sont reliées entre elles par le fabricant au niveau de zones de couture latérale (76) situées de part et d'autre, et comprenant une portion d'entrejambe (64) présentant le corps absorbant (10), qui s'étend dans la direction longitudinale de produit (14) entre la portion ventrale (60) et la portion dorsale (62) et ponte celles-ci et qui est rapportée de manière inamovible sur la portion ventrale (60) et sur la portion dorsale (62) dans une zone de chevauchement respective, dans lequel ladite portion ventrale (60), ladite portion dorsale (62) et ladite portion d'entrejambe (64) délimitent ensemble les ouvertures de jambe (18), dans lequel d'autres moyens d'élastification (66) sont prévus dans la portion ventrale (60) et dans la portion dorsale (62), qui s'étendent dans la direction transversale de produit (16) et élastifient en nappe lesdites portions ventrale (60) et dorsale (62), et dans lequel la portion d'entrejambe (64) forme ladite structure stratifiée (2) comprenant le corps absorbant (10), dans lequel, en particulier, le premier et/ou le troisième enduit de colle (24, 36) s'étendent dans la direction longitudinale de produit (14) jusqu'à des extrémités longitudinales de la structure stratifiée (2) comprenant le corps absorbant (10).

18. Procédé de fabrication d'un article hygiénique ou d'une structure stratifiée (2) comprenant un corps absorbant (10) d'un article hygiénique selon l'une ou plusieurs des revendications précédentes, dans lequel les moyens d'élastification (12) allongés sont fixés le long d'au moins une partie des zones marginales (20) délimitant les ouvertures de jambe ou découpes de jambe (18) des deux côtés, entre des portions de matériau plat (4, 6) de l'article hygiénique, comprenant les caractéristiques suivantes:
- amener un non-tissé (8) sans fin formant ladite portion de matériau plat (6) tournée vers le corps, dans une direction de machine (13) qui correspond à la direction longitudinale de produit (14) ultérieure,
- appliquer de manière continue et sur toute la surface le premier enduit de colle (24) sur la face opposée au corps (22) du non-tissé (8) dans les zones marginales (20) des deux côtés dans la direction de machine (13),
- appliquer de manière cadencée et sur toute la surface ledit deuxième enduit de colle (26) dans la direction de machine (13) sur la face opposée au corps du non-tissé (8) dans les zones marginales (20) des deux côtés dans la direction transversale de produit (16) à l'extérieur du premier enduit de colle (24) de telle sorte que le deuxième enduit de colle (26) recouvre en partie ledit premier enduit de colle (24) dans la direction transversale de produit (16) ou est immédiatement contigu au premier enduit de colle (24) ou est espacé du premier enduit de colle (24) dans la direction transversale de produit (16), le deuxième enduit de colle (26) est appliqué de manière cadencée de telle sorte qu'il est plus court que le premier enduit de colle (24) dans la direction longitudinale de produit (14) vers l'avant et vers l'arrière, donc se termine en amont de celui-ci.
- amener, dans la direction de machine (13), une bande de matériau plat sans fin formant ladite portion de matériau plat (4) opposée au corps,
- amener, dans la direction de machine (13), lesdits moyens d'élastification (12) des deux côtés entre le non-tissé (8) et la bande de matériau plat opposée au corps, au moins par sections, au niveau des premier et deuxième enduits de colle (24, 26),
- réunir le non-tissé (8) sans fin et la bande de matériau plat opposée au corps en interposant les moyens d'élastification (12), et fixer les moyens d'élastification (12) par les premier et deuxième enduits de colle (24, 26),
- déélastifier les moyens d'élastification (12) dans une zone (28) qui est restée exempte de colle au cours du cadencement du deuxième enduit de colle (26) dans la direction longitudinale de produit (14).

19. Procédé selon la revendication 18, **caractérisé en outre par** une application sur toute la surface d'un troisième enduit de colle (36) dans la direction de machine (13) sur la face opposée au corps (22) du non-tissé (8) dans les zones marginales (20) des deux côtés, dans la direction transversale de produit (16), à l'extérieur du deuxième enduit de colle (26) et à l'extérieur des moyens d'élastification (12) des deux côtés, en particulier que ledit troisième enduit de colle (36) est appliqué de manière continue dans la direction de machine (13).

20. Procédé selon l'une ou plusieurs des revendications 18 à 19, **caractérisé par le fait que**, pour fixer les moyens d'élastification (12), on n'utilise que les premier et deuxième enduits de colle (24, 26) et que la bande de matériau plat opposée au corps reste exempte de revêtement dans cette zone.

21. Procédé selon l'une ou plusieurs des revendications 18 à 20, **caractérisé par le fait que**, pour appliquer les premier et deuxième enduits de colle (24, 26), en particulier aussi pour appliquer le troisième enduit de colle (36), on utilise un dispositif d'application de colle (42, 44) qui délivre de la matière adhésive par des orifices de sortie de colle en particulier en forme de fente, en contact le plus proche avec le non-tissé.

22. Procédé selon l'une ou plusieurs des revendications 18 à 21, **caractérisé par le fait que**, après avoir réuni le non-tissé (8) et la bande de matériau plat opposée au corps, des coupes de contour menées de part et d'autre dans la direction de machine (13) sont réalisées afin de former les ouvertures de jambe ou découpes de jambe (18).

23. Procédé selon l'une ou plusieurs des revendications précédentes 18 à 22, **caractérisé par le fait que** l'on réalise ainsi une bande sans fin de structures stratifiées (2) comprenant chacune un corps absorbant (10), qui comprend la bande de matière plat opposée au corps et le non-tissé (8) tourné vers le corps ainsi qu'une suite sans fin de corps absorbants (10) transportés successivement dans la direction de machine (13) qui sont transportés et disposés entre la bande de matière plat opposée au corps et le non-tissé (8) tourné vers le corps, et qu'une coupe d'individualisation est réalisée transversalement à la direction de machine (13) pour former les structures stratifiées (2), que les structures stratifiées (2) individualisées sont tournées de 90° et sont mises en agencement pontant avec une bande de portion ventrale (60) et une bande de portion dorsale (62) et sont fixées de manière inamovible dans la zone de chevauchement avec la bande de portion ventrale (60) et la bande de portion dorsale (62).
